# EUROPEAN PATENT APPLICATION

(11) **EP 1 111 055 A1**
(43) Date of publication of application: **27.06.2001**
(21) Application number: 99403079.9
(22) Date of filing: 08.12.1999
(51) Int. Cl.: C12N 15/53, C12N 9/08, A61K 38/44, C12N 15/79, C12N 15/74, C12N 15/67, C12P 21/02

(54) **Plant enzymes with phospholipid hydroperoxide glutathione peroxidase activity, their analogues and their use**

(71) Applicant: Vetigen, 75015 Paris (FR)
(72) Inventor: Strosberg, Arthur Donny, 75015 Paris (FR); Eshdat, Yuval, 76468 Rehovot (IL)
(74) Representative: Mouget-Goniot, Claire

(57) **Abstract**

Plant enzymes with phospholipid hydroperoxide glutathione peroxidase activity PHGPx, their analogues, their methods of engineering and their use in antioxidant cosmetic or pharmaceutical dermatological compositions and for aesthetic treatment, to prevent skin cells damage, ageing and/or necrosis.

## Description

The present Invention relates to plant enzymes with phospholipid hydroperoxide glutathione peroxidase (PHGPx) activity, their analogues and their use.

Glutathione peroxidases (GPx)s are a family of multiple isoenzymes which catalyse the reduction of hydrogen peroxide, organic hydroperoxides and lipid peroxides by reduced glutathione, and help to protect the cells against oxidative damage (Flohé L. *et al.,* (1984), Methods Enzymol. **105:** 114-121; Flohé L. (1982), in: Lipid Peroxides in Biology and Medicine (Yagi, K, ed) pp. 149-159, Academic Press, New York).

Four distinct major groups of these isoenzymes, all containing the rare selenocysteine residue in their active site (Bock A. *et al.,* (1991), TIBS **16:** 463-467 ; Sunde R. A., (1990), Annu. Rev. Nutr. **10:** 451-474) have so far been characterised in mammalians. These groups of isoenzymes which differ in their structure, substrate specificity and tissue distribution (Chu F. F. *et al.,* (1993), J. Biol. Chem. **268,** 2571-2576 are :
- the cellular and cytoplasmic glutathione peroxidase (c-GPx) (Rotruck J. T. *et al.,* (1973), Science **179,** 588-590),
- the plasma glutathione peroxidase (Yamamoto Y. *et al.,* (1993), Arch. Biochem. Biophys. **305,** 541-545; Esworthy R. S. *et al.,* (1993), Arch. Biochem. Biophys. **307:** 29-34),
- the cytosolic gastrointestinal glutathione peroxidase (Chu F. F. *et al.,* (1993) reference cited), and
- the phospholipid hydroperoxide glutathione peroxidase (PHGPx) (Maiorino M. *et al.,* (1990), Meth. Enzymol. **186:** 448-457 ; Brigelius-Flohé R. *et al.,* (1994), J. Biol. Chem. **269**: 7342-7348 ; Sunde R. A. *et al.,* (1993), Biochem. Biophys. Research Comm. **193:** 905-911; Esworthy R. S. *et al.,* (1994) Gene **144:** 317-318 ; Maiorino M *et al.,* (1995), Biol. Chem. Hoppe-Seyler **376:** 651-660).

Recently the Inventors afforded, for the first time, the isolation of a plant analogue of one of these enzymes, the citrus phospholipid hydroperoxide glutathione peroxidase (Eshdat Y. *et al.,* (1997), Physiol. Plant. **100:** 234-240 ; Faltin Z. *et al.* (1998), Phys. Plant, **104,** 741-746), designated as Cit-PHGPx and called Citrus Stress-Associated Protein (Cit-SAP); its partial structural analysis revealed homology to glutathione peroxidase (Ben-Hayyim G. *et al.,* (1991) Proc. 15th International Congress of Biochem. 90).

Two genes, from tobacco and citrus (Criqui M. C. *et al.,* (1992), Plant Mol. Biol. **18:** 623-627 ; Holland D. *et al.,* (1993), Plant Mol. Biol. **21:** 923-927) were isolated and showed significant sequence homology (52%) towards mammalian PHGPx (Brigelius-Flohé R. *et al.,* (1994), reference cited ; Sunde R. A. *et al.,* (1993), reference cited; Esworthy R. S. *et al.,* (1994) reference cited). In both cases, the nucleotide sequence of the genes suggests that the amino acid sequence of the plant protein does not contain a selenocysteine residue in its presumed active site, in contrast to the animal enzyme, but a cysteine residue instead. In a recent study, recombinant Cit-SAP that was highly expressed in transformed *E. coli,* was purified by High Performance Liquid Chromatography, and confirmed this assumption that cysteine, and not selenocysteine, is the catalytic residue of the plant PHGPx (Faltin Z. *et al.* (1998) (reference cited).

The citrus gene, designated *csa,* was isolated by the Inventors from cultured citrus cells that were acclimated to high levels of salt, and its protein product, Cit-SAP (Cit-PHGPx), was initially purified in its denatured form by two-dimensional gel electrophoresis (Ben-Hayyim G. *et al.,* (1993), Plant Sci. **88,** 129-140) and its partial amino acid sequence revealed identity with that deduced from the nucleotide sequence of *csa* (Holland D. *et al.,* (1993), reference cited). Cit-SAP was found to be a cytosolic protein present in all citrus plant organs tested, and its level is increased in cultured cells exposed to NaCl and in citrus plants upon irrigation with salt (Ben-Hayyim G. *et al.,* (1993) reference cited). In line with its sequence homology to PHGPx, the recombinant protein was shown to provide increased tolerance to oxygen radicals in transformed *E. coli* cells expressing Cit-SAP (Holland D. *et al.,* (1994), FEBS Lett. **337:** 52-55).

In a later study the Inventors have partially purified Cit-SAP from cultured citrus cells by affinity chromatography, and established its ability to catalyse specifically the reduction of phosphatidylcholine hydroperoxide by GSH (Beeor-Tzahar T. *et al.,* (1995), FEBS Letter, **366,** 151-155).

They have also partially purified glutathione S-transferase (GST) and confirmed that it also performed PHGPx activity in addition to its transferase activity as shown before by Marks (Annu. Rev. Plant Physiol. Plant Mol. Biol. (1996), **47**, 127-158).

In the recent years, a few genes which encode homologous PHGPx were isolated from several plants (Depege N. *et al.,* (1998), Eur. J. Biochem. **253:** 445-451 ; Roeckel-Drevet P. *et al.,* 1998, Physiol. Plant. **103:** 385-394 ; Sugimoto M. *et al.* (1997), Genes Genet. Syst. **72:**811-816; Sugimoto M *et al.* (1997) Biosci. Biotech. Biochem. **61:** 1379-1381).

Eshdat Y. *et al.,* (1997) (cited reference), using Cit-PHGPx antibodies, have identified PHGPx in various organs of *Aloe arborescens* and *Aloe vera.*

As stated before, a substantial difference between the Cit-SAP and the animal GPx family members lies in the amino acid sequence of their respective catalytic site.

While the mammalian PHGPx genes were found to include the codon TGA for the rare amino acid residue selenocysteine (Sec) as their catalytic residue (Bock A. *et al.,* (1991) Mol. Microbiol. **5:** 515-520), the codon for the corresponding active site amino acid in Cit-SAP is TGT (Holland D. *et al.,* (1994) FEBS Lett. **337:** 52-55), which encodes a cysteine (Cys) residue.

Since TGA is normally a stop, a stem-loop configuration (SECIS) is needed to enable identification of this codon for Sec. In the eukaryotes, the SECIS is located downstream to the 3' end of the Open Reading Frame (ORF) region. In prokaryotes, the SECIS is part of the ORF and is adjacent downstream to the TGA encoding Sec.

This difference in their catalytic site is considered to be responsible for the much lower rate of activity of the Cit-SAP compared to that of the animal enzyme (Beeor-Tzahar T. *et al.,* (1995) reference cited ; Faltin Z. *et al.,* (1998) reference cited). Indeed, Cys differs from Sec in its higher pKₐ and its lower reductive power under physiological conditions, whereas the selenol is characterized by its great polarisability of the Se containing group.

Formation of oxyradicals in cells may result in peroxidation of lipids and phospholipids that comprise the cell membrane, leading to the membrane destruction and inhibition of DNA replication. Skin cells are mostly exposed to different environmental stresses, such as UV radiation, ⁶⁰Co gamma-radiation, tropospheric ozone, hydration, oxidative stress produced by xenobiotics, and others, which may produce such hazardous reactive oxygen species (ROS). The cascade reaction of these ROS, ending in modification of the natural membrane phospholipids into phospholipid hydroperoxides, even in upper skin layers, could trigger inflammatory responses in adjacent skin layers, affect the barrier function of the stratum corneum and the epidermal function (Thiele J. J. *et al.,* (1997), Biol. Chem. **378:** 1299-1305) and mediate cancer, premature aging and necrosis of the skin (Brenneisen P. *et al.,* (1998), J. Biol. Chem. **273** : 5279-5287 and references within). Formation of ROS in skin tissue was found to be enhanced also in events of cutaneous injury, which can cause severe tissue damage (Steiling H. *et al.,* (1999), Exp. Cell Res. **247:** 484-494).

This is therefore why a variety of compounds, termed as antioxidants, are widely used in cosmetic and dermatological products (Steenvoorden D. P. T. *et al.,* (1997), J. Photoch. Photobio. **B 41:** 1-10 and references within). Since prevention of the formation of ROS (e.g. by using anti-sun creams) is never absolute, and since the skin cells defensive mechanism was shown to be less efficient after ROS-producing stress such as UV radiation (ref. 9-16 in Steenvoorden D. P. T. *et al.,* (1997) reference cited), these antioxidants are aimed at scavenging the ROS immediately following their formation, prior to their reaction with biologically vital compounds of the cells. Among these antioxidants are Vitamin E (α-tocopherol), glutathione, Vitamin C (ascorbate), β-carotene, and other. These materials, alone or together with a few enzymatic (including superoxide dismutase (SOD), catalase and cGPx) and non-enzymatic protective systems that already exist in the cell, aim at neutralising the ROS either by reacting with them directly, or by converting them into hydroperoxide compounds (like hydrogen peroxide) that will be neutralised later into stable, non-reactive, compounds (such as water).

A major harm to skin and other cells can therefore happen, if the present strategy fails to either neutralise directly the ROS, or reduce the H₂O₂ into water. An excess of H₂O₂ in the cell will lead to formation of highly reactive ROS in the form of hydroxyl radicals (OH.), which by reacting with phospholipids will turn the latter into phospholipid hydroperoxides, which will lead to the skin cells damage as mentioned above. It has already been shown that most promising results in protecting the skin against ROS damage is to combine several antioxidant compounds resulting in synergism of the protective effects (Steenvoorden D. P. T. *et al.,* (1997) reference cited).

Despite this, there is still a need for an improvement of this protective effect against ROS skin damage.

Therefore it is a goal of the present Invention to provide means for repairing the peroxidation damage caused by failure of the antioxidative defense system to prevent hydroxyl radical formation and its reaction with lipids and their derivatives, said means serving as a second line of defense against skin cell tissue destruction.

In satisfying these objectives, the present Invention provides isolated plant phospholipid hydroperoxide glutathione peroxidase (PHGPx) and their analogues.

The following definitions are provided to facilitate understanding of certain terms used frequently herein.

« PHGPx » refers among others, generally to a polypeptide having the amino acid sequence set forth in SEQ ID N° 2 (Cit-PHGPx or Cit-SAP) or an allelic variant thereof. According to the present Invention, the plant-PHGPx may be used in the form of enriched plant extracts, partially or completely purified enzyme, recombinant enzyme in *E. coli* or other cell types, and genetically engineered modified enzyme, all having equivalent or improved protecting effect against ROS skin damage.

«PHGPx activity» refers to the biological function of Cit-PHGPx including similar activity or improved activities or these activities with decreased side-effects.

«PHGPx gene» refers to a polynucleotide having the sequence setforth in SEQ ID N° 1 or an allelic variant and /or their complement.

« Isolated » means « altered by the hand of man » from the natural state.

« Polypeptide » refers to any peptide or protein comprising two or more amino acids joined to each other by peptide bonds. They may contain amino acids other than the 20 classical gene-encoded amino acids and include modified sequences, either by natural process or by chemical modification or recombinant techniques or produced by a combination of these last two methods, which are well known in the art.

« Analogues » as the term is used herein is a natural or not natural polynucleotide or polypeptide that differs from the reference polynucleotide or polypeptide respectively but retains essential properties.

« Identity » is a measure of the identity of nucleotides sequence or amino acids sequence which is determined by *per se* known techniques. Methods commonly employed to determine identity and similarity are codified in computer programs, as for example the gap program of GCG (Wisconsin Package Version 10., Genetics Computer Group (GCG), Madison, Wisconsin). As an illustration, if a polynucleotide has at least 95 % identity to a reference sequence, it is intended that its sequence may include up to five point mutations (deletion, substitution or insertion) per each 100 nucleotides of the reference nucleotide sequence. These mutations may occur at the terminal positions of the reference sequence or anywhere between those terminal positions. Similarly, if a polypeptide has at least 95 % identity to a reference sequence, it is intended that its sequence may include up to five amino acids alteration (deletion, substitution or insertion) per each 100 amino acids of the reference amino acids sequence. These mutations may occur at the terminal positions of the reference sequence or anywhere between those terminal positions.

« Similarity » is a measure of the similarity of nucleotides sequence or amino acids sequence which is determined by *per se* known techniques. Methods commonly employed to determine identity and similarity are codified in computer programs, as for example the gap program of GCG (Wisconsin Package Version 10., Genetics Computer Group (GCG), Madison, Wisconsin). As an illustration, if a polypeptide has at least 95 % similarity to a reference sequence, it is intended that its sequence may include up to five amino acid substitutions per each 100 amino acids of the reference polypeptide sequence. Typical conservative substitutions are among Ala, Leu, Val and Ile; among Ser and Thr; among the acidic residues Asp and Glu; among Asn and Gln and among the basic residues Lys and Arg.

In one aspect, the present Invention relates to isolated plant PHGPx and their analogues comprising an amino acid sequence which is at least 60 % identical or similar to the amino acid sequence of SEQ ID N° 2 over its entire length, with the proviso that they are not SEQ ID N° 2.

In one specific embodiment, the isolated plant PHGPx is selected from the group consisting of PHGPx isolated from *Aloe arborescens* (SEQ ID N° 4 and SEQ ID N° 6) and PHGPx isolated from *Aloe vera* (SEQ ID N° 8 and SEQ ID N° 10).

In still another embodiment, the recombinant plant PHGPx is a recombinant plant PHGPx, wherein the cysteine residue in the active site, at a position homologous to position 41 in SEQ ID N° 2, is replaced by a selenocysteine residue.

In a specific embodiment, the analogue is a recombinant plant PHGPx which is selected from the group consisting of SEQ ID N°11, SEQ ID N°12, SEQ ID N° 13, SEQ ID N° 14, SEQ ID N° 15, SEQ ID N° 16, SEQ ID N° 17, SEQ ID N° 18, SEQ ID N° 19 and SEQ ID N° 20.

The PHGPx according to the Invention may be in the form of the « mature » protein or may be part of a larger protein, such as fusion protein.

Another aspect of the Invention is to relate to PHGPx polynucleotides comprising a sequence which is at least 45 % identical or similar to sequence SEQ ID N° 1 encoding the Cit-SAP sequence of SEQ ID N° 2 over its entire length, with the proviso that these polynucleotides are not SEQ ID N° 1.

The Invention also provides polynucleotides which are complementary to such PHGPx polynucleotides.

In a specific embodiment according to the present Invention, the polynucleotide is selected from the group consisting of SEQ ID N° 3 *(alarp 1),* SEQ ID N° 5 *(alarp 2),* SEQ ID N° 7 *(alvep 1)* and SEQ ID N° 9 *(alvep 2).*

Another aspect of the Invention provides a method for engineering, in a prokaryotic or eukaryotic cell, PHGPx containing a selenocysteine instead of a cysteine, at their active site, at a position homologous to position 41 in SEQ ID N° 2.

Indeed the Inventors introduce a modified prokaryotic SECIS *(selenocystein insertion sequence)* in the coding sequence of the *csa* gene or of an analogous gene, in order to produce in prokariotic cells, like for example *E. coli,* a plant PHGPx active enzyme, possessing a selenocysteine as a catalytic residue.

The modified SECIS inserted in the *csa* gene or in an analogous gene were designed so as to minimize changes in the Cit-SAP amino acid sequence while allowing an efficient interaction between the mRNA stem-loop structure and the other bacterial factors involved in the selenocysteine incorporation, including the tRNA^{Sec}. Modifications of the primary structure should be minimal in order to retain the enzymatic activity.

In a specific embodiment, the method for engineering plant PHGPx containing a selenocysteine instead of a cysteine at their active site, in a prokaryotic cell, is performed by introducing an appropriate stem-loop (SECIS) in a gene coding for a plant PHGPx by site specific mutagenesis comprising the steps of:
- performing amplification of the sequence containing said gene coding for a plant PHGPx in two fragments: (i) one fragment amplified by two primers: one, chosen from the sequence coding said gene, containing the anticodon corresponding to the catalytic residue (Cys or Sec), and one located in the plasmid carrying said gene, (ii) one fragment amplified by two primers: one containing the sequence of the stem-loop structure to be introduced and one located in the plasmid carrying said gene,
- digesting the appropriate DNA fragments with appropriate restriction enzymes,
- performing a ligation, and
- transfecting competent prokaryotic cells.

In an other specific embodiment, for engineering of Cit-PHGPX^{sec}, the sequence containing *csa* gene is pARO1 described by D. Holland *et al.* (1994) (reference cited) which contains the ampicillin-resistance gene, ColE1 ori and the promoter *lac,* and the primers are selected from the group consisting of SEQ ID N° 47 and SEQ ID N° 48 associated to a primer of SEQ ID N° 46 and a primer coding for the mRNA stem-loop structure selected from the group consisting of SEQ ID N° 49, SEQ ID N° 50 and SEQ ID N° 51, said primer being associated to a primer of SEQ ID N° 45, and the digesting step of the first fragment is performed with AlwNI enzyme and HpaI enzyme and the digesting step of the second fragment is performed only with AlwNI enzyme.

In an other specific embodiment of the engineering method, the prokaryotic cell is *E. coli.*

The Inventors also introduce an eukaryotic SECIS *(selenocystein insertion sequence)* in the non-coding sequence of the *csa* gene or of an analogous gene, in order to produce a plant PHGPx active enzyme possessing a selenocysteine as a catalytic residue, in eukaryotic cells like COS cells or yeast cells.

In a specific embodiment, the method for engineering plant PHGPx containing a selenocysteine instead of a cysteine at their active site, in an eukaryotic cell, is performed by converting TGT codon to TGA and fusing the 3'UTR of plant PHGPx containing the SECIS element to *csa,* in the following steps :
- converting TGT codon to TGA by site directed mutagenesis,
- synthesising of the 3'UTR of pig PHGPx by annealing 6 synthetic oligonucleotides,
- fusing of the 3'UTR of pig PHGPx to either the 3' end of the open reading frame of *csa* or the 3' end of *csa,* by PCR,
- cloning in mammalian and yeast vectors and,
- transforming competent eukaryotic cells.

In an other specific embodiment of the engineering method, the eukaryotic cells are COS cells.

Another aspect of the Invention is to relate to cosmetic compositions or pharmaceutical dermatological compositions to prevent lipid and phospholipid modification due to their peroxidation, which leads to skin cells damage, ageing and/or necrosis, said compositions comprising at least one active ingredient selected from the group consisting of isolated plant PHGPx or analogues thereof, and plant enzymes with PHGPx activity or analogues thereof, as active ingredient with antioxidant activity.

In a specific embodiment, the PHGPx is selected from the group consisting of SEQ ID N° 2, SEQ ID N° 4, SEQ ID N° 6, SEQ ID N° 8, SEQ ID N° 9, SEQ ID N° 10, SEQ ID N° 11, SEQ ID N° 12, SEQ ID N° 13, SEQ ID N° 14, SEQ ID N° 15, SEQ ID N° 16, SEQ ID N° 17, SEQ ID N° 18, SEQ ID N° 19 and SEQ ID N° 20.

In another specific embodiment, the plant enzyme with PHGPx activity is gluthatione S-transferase (GST).

In still another embodiment, the cosmetic or pharmaceutical dermatological compositions may comprise at least one further antioxidant selected from the group consisting of Vitamin E, Vitamin C, β-carotene, glutathione and others commonly used antioxidants.

Another aspect of the Invention relates to the use of compounds selected from the group consisting of plant PHGPx or analogues thereof and of plant enzymes with PHGPx activity or analogues thereof, for the manufacture of an antioxidant cosmetic or pharmaceutical dermatological composition to prevent lipid and phospholipid modification due to their peroxidation, which leads to skin cells damage, ageing and/or necrosis.

The compositions according to the present invention may be used to protect, treat or prevent the damage caused by free radicals to the skin. They may be used to repair and reverse skin damage due to environmental stress and to retard skin ageing resulting in lost of elasticity, wrinkles and age spots.

Numerous studies showed that the natural phospholipids, which are the main components of all biological membranes, reduce water loss, sunburns, pollution, effects and simply formulate skin friendly cosmetics (Isabello S. *et al..*(1999)*,* IBC meeting "The skin -its problem and protection").

Consequently, another aspect of the Invention relates to the use of compounds selected from the group consisting of plant PHGPx or analogues thereof and from plant enzymes with PHGPx activity or analogues thereof, to protect phospholipids used in cosmetic compositions or pharmaceutical dermatological compositions, against phospholipids oxidation.

Melanin pigment level is dependent on oxidative reactions and lipoxigenase activity, among other factors (Rosei M. A. *et al.* (1998), *Int. J. Biochem. Cell Biol.,* **30,** 457-463 ; Blarzino C. *et al.* (1999), *Free Radical Res.* **26,** 446-453) and PHGPx could be capable of reducing the melanin level and provide skin lightening.

Consequently, another aspect of the Invention relates to the use of compounds selected from the group consisting of plant PHGPx or analogues thereof, and from plant enzymes with PHGPx activity or analogues thereof, as skin-lightening supporting agents in cosmetic compositions or pharmaceutical dermatological compositions.

In the cosmetic compositions or pharmaceutical dermatological compositions according to the Invention, plant-PHGPx or other plant enzymes with PHGPx activity, may be used in the form of enriched plant extracts, partially or completely purified enzyme, recombinant enzyme in prokaryotic or eukaryotic cell types, and genetically engineered modified enzyme.

In yet another aspect of the Invention, there is provided a method of aesthetic treatment of a human to prevent lipid and phospholipid modification due to their peroxidation, which leads to skin cells damage, ageing and/or necrosis, which comprises administering to said human at least one compound selected from the group consisting of isolated plant PHGPx, analogues thereof, and other plant enzymes with PHGPx activity, said plant PHGPx being preferably selected from the group consisting of SEQ ID N° 2, SEQ ID N° 4, SEQ ID N° 6, SEQ ID N° 8, SEQ ID N° 9, SEQ ID N° 10, SEQ ID N° 11, SEQ ID N° 12, SEQ ID N° 13, SEQ ID N° 14, SEQ ID N° 15, SEQ ID N° 16, SEQ ID N° 17, SEQ ID N° 18, SEQ ID N° 19 and SEQ ID N° 20 and said other plant enzymes with PHGPx activity being glutathione S-transferase.

The following drawings form part of the present specification and are included to further demonstrate certain aspects of the present Invention. The Invention may be better understood by reference to one or more of these drawings combined with the detailed description of specific embodiments presented herein :

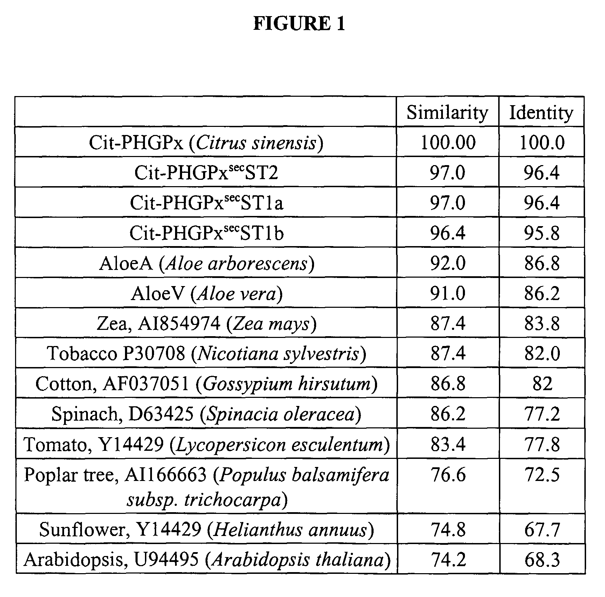

**Figure 1** illustrates the similarity and identity between the Cit-PHGPx, the engineered Cit-PHGPx^{Sec} and other plant PHGPx. Comparison of each of the sequences to the Cit-PHGPx was done using the GCG (Wisconsin Package Version 10., Genetics Computer Group (GCG), Madison, Wisconsin).

**Figure 2** illustrates the presence of PHGPx in *Aloe arborescens* and *Aloe vera* in various organs according to example 1. "CB staining" is Comassie Blue staining, "WB" is Western Blott, "A" is *Aloe Arborescens, "V"* is *Aloe vera,* "Sap" correspond to parenchymal gel and "CSA+ *E. coli" is* used as control and corresponds to *E. coli* expressing Cit-PHGPx.

**Figure 3** is a schematic representation of cloning of *Aloe urborescens* genes and *Aloe vera* genes according to example 2.

**Figure 4** shows the alignment of all the 3' RACE cloned sequences according to example 2.

**Figure 5** shows the alignment of all the 5' RACE cloned sequences according to example 2.

**Figure 6** shows the expression of AloA-PGPHx in *alarp1* transformed BL(21)DE3 *E. coli* cells according to example 4. "CB staining" is Comassie Blue staining. 343 and 344 are specific Cit-SAP antibodies prepared according to example 1.1.5.

**Figure 7** illustrates the SECIS mRNA structures introduced in the *csa* coding sequence according to example 5.

**Figure 8** shows the strategy used for insertion of the SECIS motifs in *csa* gene in pARO1, according to the method disclosed in example 5. « Amp » represents the ampicillin-resistance gene, « ColE1 ori » represents the ColE1 origin, *«csa »* represents the citrus gene, « prST -», «prST+», «prST TGT/A» and « prST1a,b or 2 » represent primers and « LacZ » represents the Lac gene.

**Figure 9** shows the aligned sequences from Cit-SAP, human pGPx and ST mutants ; « # » corresponds to a selenocysteine residue, « Y' » and « F' » correspond to aromatic amino acids, « S" » and « T" » correspond to hydroxy amino acids ; amino acids homologous to Cit-SAP are shaded.

**Figure 10** shows the effect of the mutagenesis on the level of expression and/or stability of the mutants compared to those of the Cit-SAP according to example 5.2.2. (**a**) protein extracts; (**b**) PHGPx activity. A unit is 1 nmole of NADPH oxidized/min at room temperature with respect to total extract protein.

**Figure 11** shows the expression of mutants containing. a selenocysteine as catalytic residue according to example 5.2.3. (**a**) Red Ponceau analysis of protein extracts after SDS-PAGE (**b**) Western-blot analysis of PHGPx in protein extracts.

**Figure 12** shows the schematic representation of *csa* constructs fused to mammalian 3'UTR of PHGPx (SL fragment) and fusion strategy as disclosed in example 6.

**Figure 13** shows the protein expression of CSA^{TGT} and CSA^{TGA} fused to mammalian 3'UTR of PHGPx in yeast according to example 6; in Western blot of SDS-PAGE yeast extracts, lane 1 corresponds to *csa E. coli,* lane 2 corresponds to B^{TGT}, lane 3 corresponds to *csa*^{TGT}, lane 4 corresponds to A^{TGT}, lane 5 corresponds to pYES2, lane 6 corresponds to A^{TGA}, and lane 7 corresponds to B^{TGA}.

**Figure 14** shows the expression of Cit-PHGPx in *csa*^{*TGT*} and *csa*^{*TGA*} transfected COS cells according to example 6. "C" corresponds to control cells ; "1" corresponds to Cit-PHGPx^{Cys} and ; "2" corresponds to Cit-PHGPx^{Sec}.

The following examples are included to demonstrate preferred embodiments of the Invention. It should be appreciated by those skilled in the art that the techniques disclosed in the examples which follow can be considered to constitute preferred modes for the practice of the Invention. However those skilled in the art should, in light of the present disclosure, appreciate that many changes can be made in the specific embodiments which are disclosed and still obtain a like or similar result without departing form the concept, spirit and scope of the Invention.

### Example 1 : Presence of PHGPx in Aloe arborescens and Aloe vera various organs

### 1.1. Material and methods:

1.1.1. Plant material: *Aloe arborescens* plants were taken from the garden of Volcani Center, Bet Dagan (Israel) and *Aloe vera* plants *(Aloe barbadensis* Miller) were taken from Moshav Ein Yahav originally brought from Texas (USA).
1.1.2. Crude protein extract of plant material: protein extracts were prepared from roots, upper stalk and leaf of *Aloe arborescens* and *Aloe vera.*
   Roots and leaves were washed with water. The leaf was separated into the leaf skin (outer rind) and sap (parenchymal gel). For visualisation of the upper stalk, theJeaves were taken off and the segment from the leaf to the first internode was used.
   Plant material (except of the parenchymal gel) frozen by liquid nitrogen was ground to fine powder with pestle and mortar and the proteins extracted with 2 x SDS extraction buffer, boiled and centrifuged for the removal of cell debris.
   The gel material was homogenised without freezing.
   The supernatant was used for protein quantification, gel electrophoresis and immunoblotting.
1.1.3. Antibody preparation: polyclonal antibody was prepared as described by Ben-Hayyim *et al.,* Plant Sci. (1993), **88**, 129-140).
   Two specific antibodies were produced:
   - antibody 344 was raised against the N-terminal peptide located between amino acids 1-21, and
   - antibody 343 was raised against the peptide 71-89 of the protein.

   A cysteine residue was added to the peptides and the peptides were acetylated and amidated. The peptides were coupled to KLM (Keyhole Limpet Hemocyanin) and the emulsified KLH-conjugate was injected to rabbits for antibody production.
   The polyclonal antiserum was affinity purified by a chromatography column containing the N-terminal peptide covalently coupled to activated Sepharose-4B.
1.1.4. SDS PAGE and Western blotting: SDS PAGE and Western blotting was done as previously described by Ben-Hayyim *et al.,* (1993) (reference cited).
   Western blots were reacted with polyclonal antibody and 344 antibody raised against the N-terminal peptide of Cit-PHGPx at a concentration of 100 ng/ml. The blots were stained with alkaline phosphatase conjugated to the second antibody.

### 1.2. Results:

### 1.2.1. The presence of PHGPx in Aloe arborescens and Aloe vera various organs:

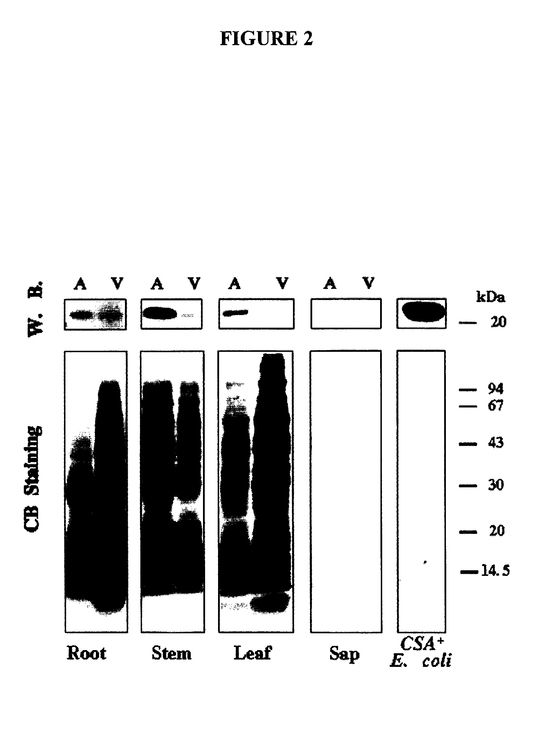

The results are given in Figure 2.

PHGPx can be detected in all organs tested, except for the inner leaf gel (sap).

The highest amount was found in the upper stem, where high cell division was described (Kawai K. *et al.,* (1993), Phytotherapy Res. **7,** S5-S10).

*Aloe vera* seems to produce much less PHGPx that is cross reacting with 344 Ab than *Aloe arborescens.* Approximately 60 µg of protein from the various organs and 5 µg from the gel material were loaded. At that protein level no PHGPx could be detected in the gel.

### Example 2 : Cloning of PHGPx cDNA's from Aloe arborescens

### 2.1. Material and methods:

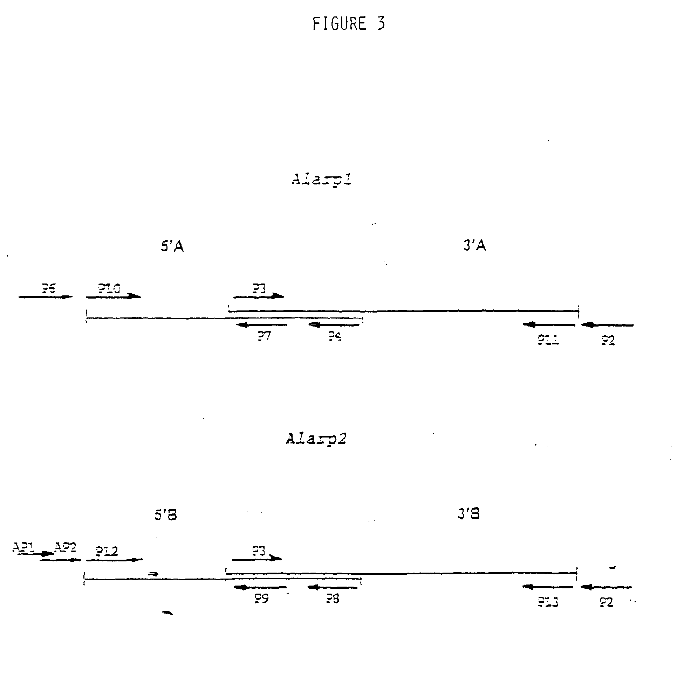

2.1.1. mRNA isolation: preparation of highly purified and full length mRNA is a critical step in gene isolation. Since the Aloe plants contain high amounts of gel material rich in polysaccharides, total RNA was extracted from the superior stalk *of Aloe arborescens* according to the method of Chang S. *et al.* (1993) (reference cited), developed for tissue containing high levels of polysaccharides and polyphenols.
   The method is based on CTAB/chloroform extraction. CTAB contains high salt concentration (2 M NaCI) and anti-oxidative agents. RNA is precipitated with LiCI and redissolved with high salt concentration (1 M NaCl) in order to eliminate polysaccharides.
   Poly(A+)mRNA was isolated from total RNA using the polyATtract kit of Promega following the instructions of the manufacturer.
2.1.2. Cloning of *Aloe arborescens:* cloning of *Aloe arborescens* cDNA was basically done using the RACE strategy (Frohman M. A. *et al.,* (1998), *Proc. Natl. Acad. Sci. USA,* **85,** 8998-9002) which include cDNA synthesis from poly(A+)mRNA and isolation of two overlapping fragments, 3' end fragment and 5' end fragment.
   3' RACE was done using a degenerate primer (SEQ ID N° 21) and a 3' poly (dT) anchored primer. Based on the sequence of the 3' fragment, the 5' end was amplified with a specific primer and a 5' anchored primer.
   The primers and their sequences are listed in Table 1 and a schematic representation of said cloning method is given in figure 3.
   a) *cDNA synthesis:* first strand cDNA was synthesised with RNAse H⁻ reverse transcriptase (Superscript II, Gibco BRL) according to the manufacturer's instructions, using 500 ng poly(A+)mRNA and 3'anchore-linked poly(dT) (Primer 1, SEQ ID N° 21). For annealing, 1 µl of 100 µM of primer was incubated with poly(A+)mRNA for 10 min at 70 °C in a final volume of 12 µl. The reverse transcriptase reaction (RT reaction) was carried out in 20 µl reaction volume containing 4 µl reaction buffer, 2 µl 0.1 M DTT, 1 µl 10 mM dNTP, 10 µl of Rasine (Promega), and 200 unit of reverse transcriptase. The reaction was incubated at 42 °C for 1 h, followed by inactivation of the enzyme at 70 °C for 15 min.
   b) 3' *RACE :* 3'end amplification was done with the forward degenerate Primer 3 (SEQ ID N° 23), and the reverse 3'anchored Primer 2 (SEQ ID N° 22). The degenerate primer was designed for the deduced amino acid sequence VNVASK/QCG. This sequence was found to be conserved in all 17 plant-PHGPx homologous sequences found by the Blast search of the GenBank database.
      Alignment of the deduced amino acid sequences was done using the Pileup program of the GCG software.
      PCR was carried out with 1 µl of 1/10 diluted cDNA, 1 µl of 20 µM of Primer 2 (SEQ ID N° 22), 1 µl of 100 µM stock of degenerate Primer 3 (SEQ ID N° 23), 1 µl of 10 mM stock of dNTP and 1 unit of Tag polymerase (Boerhinger-Mannheim). After an initial denaturation of 2 min. at 94 °C, a step program of 40 cycles was carried out which included primer denaturation at 94 °C for 20 sec, annealing at 55 °C for 30 sec and elongation at 72 °C for 1 min. Final extension at 72 °C for 7 min was done after the last cycle.
      All the following PCR reactions were done at the described conditions except for the annealing temperature and number of cycles. Denaturation for 40 sec, annealing for 1 min and final extension for 15 min were carried out when using an older model of the Thermal Cycler.
      The PCR fragment of 600 bp was purified from agarose gel with the Qiax II gel extraction kit (Qiagene) and cloned in the T/A pGEM-T Vector (Promega) according to the manufacturer's protocol.
   c) 5' *RACE:* to obtain the 5'A fragment, a specific cDNA was synthesised from *Aloe arborescens* poly(A+)mRNA and an anchored oligonucleotide was ligated to the 5'end of the cDNA according to Trout A. B. *et al.* (Proc. Natl. Acad. Sci. USA, (1992), **89**, 9823-9825). PCR was carried out with a reverse 3' specific primer and a forward complement primer of the 5' anchored oligonucleotide.
      Specific cDNA was synthesised from 500 ng poly(A) mRNA using reverse Primer 4 (SEQ ID N° 24) and 200 units Superscript II (Gibco-BRL). 1 µl of 20 µM Primer 4 (SEQ ID N° 24) was annealed to 9 µl of poly(A+)mRNA at 65 °C for 10 min. The RT reaction was carried out in 30 µl reaction volume containing 6 µl RT buffer, 4 µl 10 mM dNTP, 2 µl 0.1 M DTT and 200 units Superscript II .The reaction was incubated at 42 °C for 1h and the enzyme inactivated at 70 °C for 15 min. RNA was hydrolysed with NaOH, purified with Qiax II glass beads and precipitated.
      Primer 5 (5'anchored oligonucleotide ; SEQ ID N° 25) was phosphorylated with polynucleotide kinase (Bio-Lab) and ligated to single stranded RNA with T4 RNA ligase (Bio-Lab) in a reaction volume of 10 µl. The reaction was incubated for lh at 37 °C, following by 16 h at room temperature. PCR was carried out with reverse specific Primer 7 (SEQ ID N° 27) and 5' anchored Primer 6 (SEQ ID N° 26). 50 cycles were run at annealing temperature of 50 °C.
      To obtain the 5'B fragment the Marathon cDNA amplification kit (Invitrogen) was used. A library was constructed from *Aloe arborescens* mRNA of adaptor ligated double-stranded cDNA according to the manufacturer's protocol. Primer 8 (SEQ ID N° 28) and the Marathon AP1 primer (SEQ ID N° 37) were used for PCR reaction. Touchdown PCR was carried out according to the Marathon manual instruction: 5 cycles with annealing temperature of 72 °C, 5 cycles at 70 °C, 5 cycles at 68 °C, and 30 cycles at 60 °C. Elongation at 72 °C was carried out in all cycles. 1/10 of the reaction product was used with nested Primer 9 (SEQ ID N° 29) and Marathon AP2 (SEQ ID N° 38) in a PCR reaction of 25 cycles at annealing temperature of 60 °C.
      All PCR fragments were purified from agarose gel, cloned in pGEM-T vector and sequenced in both directions.
   d) *Full length cDNA:* generation of full length cDNA was done using the Marathon ds cDNA library and primers from 5' and 3' ends of the genes.
      Primers 10 (SEQ ID N° 30) and 11 (SEQ ID N° 31) were used to isolate *alarp1* gene. Primers 12 (SEQ ID N° 32) and 13 (SEQ ID N° 33) were used to isolate *alarp2.*
      The PCR reaction was carried out with the Expend high Fidelity polymeras kit (Boehringer-Mannheim) at annealing temperature of 55 °C and 25 cycles.

### 2.2. Results:

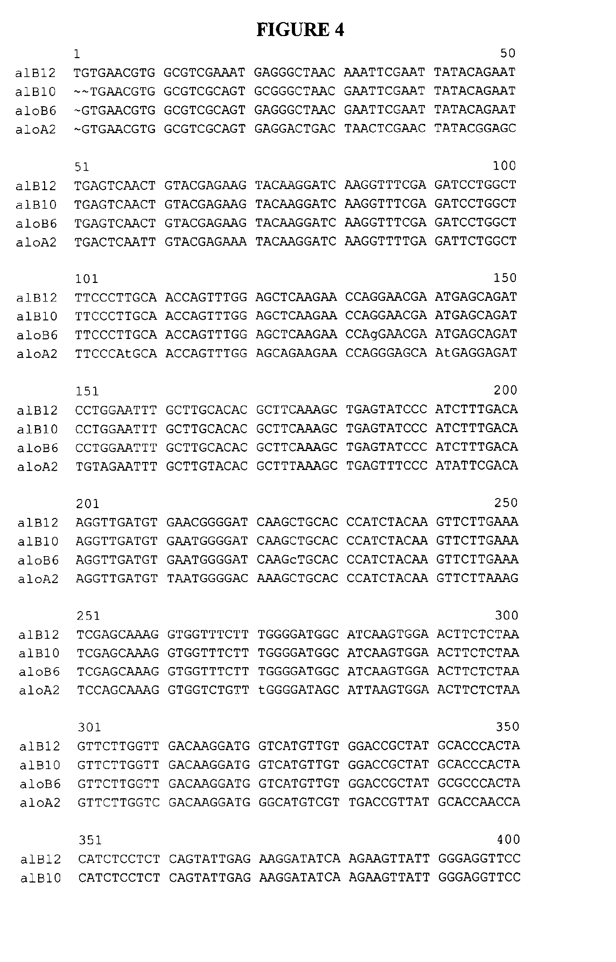

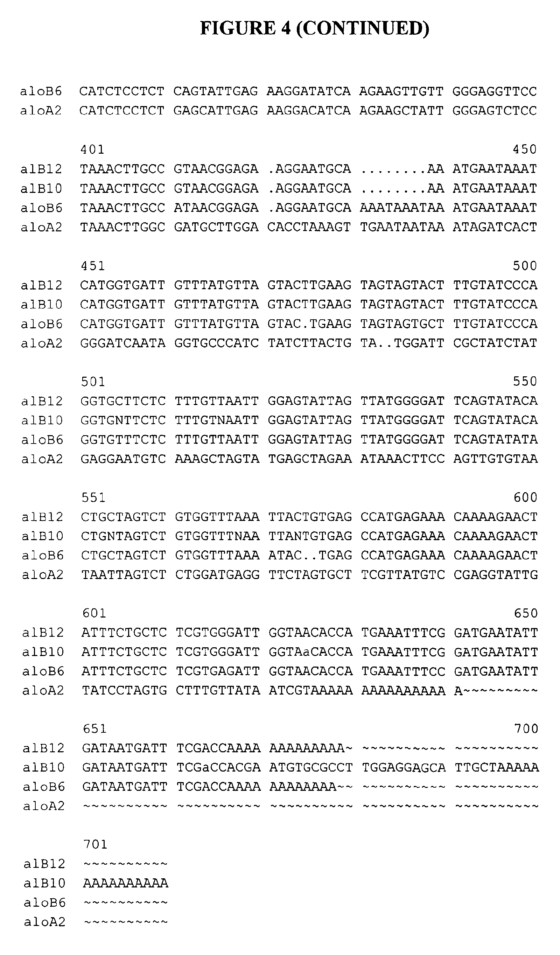

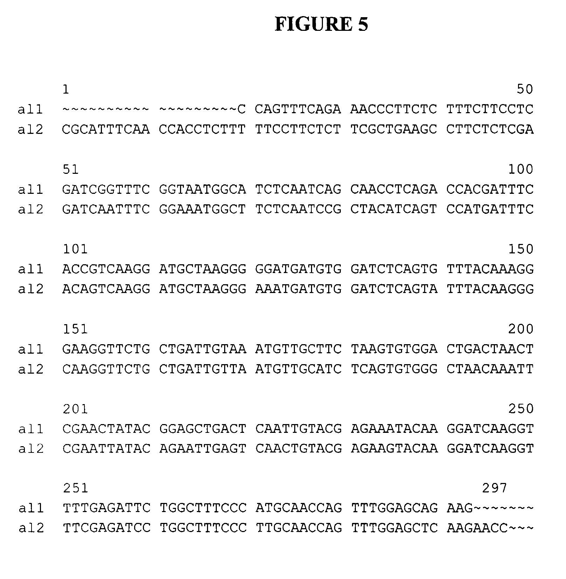

2.2.1. mRNA isolation:
   For *Aloe arborescens* 50 µg of total RNA from 1 mg of fresh tissue were recovered and about 1% of poly(A) mRNA from total RNA.
   For *Aloe vera* half of that amount were recovered.
2.2.2. *3'RACE:*
   The alignment of all the 3' RACE cloned sequences are disclosed in Figure 4.
   Two different clones were isolated and sequenced, clone A (AloA2 SEQ ID N° 39 ) and clone B (AloB6 SEQ ID N° 40).
   Using specific restriction enzymes for clones A and B, all the rest of the clones isolated could be divided either to clone A or to clone B. Two additional clones, which were cut with the specific clone B restriction enzyme, were sequenced alB10 (SEQ ID N° 41) and alB12(SEQ ID N° 42).
   A and B are completely different at the 3' untranslated region. AlB10 is longer than alB12 by 29 nucleotides at the 3' end of the sequence. They both differ from AloB6 by missing the sequence AATAAAT at nucleotides 431-438 .
2.2.3. *5'RACE:*
   The alignment of all the 5' RACE cloned sequences are disclosed in Figure 5.
   A 300 bp 5'A fragment, a11, (SEQ ID N° 43) was isolated.
   5'B fragment, a12, (SEQ ID N° 44) was isolated after a nested PCR. (Primers 9 and AP2).
   The overlapping sequence of 3'A and 5'A and 3'B and 5'B were 100% homologous.
2.2.4 *full lenght*:
   *alarp1* (SEQ ID N° 3) and *alarp2* (SEQ ID N° 5) show 75% similarity.
   *alarp1* shows 75% similarity to the Citrus gene *(csa).*
   *alarp2* shows 65% similarity to *csa.*

   The deduced amino acid sequences of *alarp1* and *alarp2* show 95% similarity. Each of the deduced amino acid sequences show 92% similarity to Cit-PHGPx .

### Example 3 : Cloning of PHGPx cDNA's from Aloe vera

### 3.1. Material and methods:

*Aloe vera* cDNA's were isolated based on the sequence of *Aloe arborescens* cDNA.

Cloning of *Aloe vera* cDNA was done on the basis of *alarp1* and *alarp2* sequences.

cDNA was synthesized from *Aloe vera* poly (A⁺) mRNA as described for *Aloe arborescens.*

PCR was carried out at 55 °C and 25 cycles with the Expend high Fidelity polymeras kit (Boehringer-Mannheim) with the following primers: primer 10, 11, 12, 13, 14 and 15 (SEQ ID N° 30 to 35 in Table 1).

In order to confirm the sequence, the reverse Primer 8 (SEQ ID N° 28) was used to re-sequence the 3'end of the fragment, and Primer 16 (SEQ ID N° 36) was used to re-sequence the 5'end of the fragment.

### 3.2. Results:

The end primer of *alarp1* (Primers 10 of SEQ ID N° 30 and 11 of SEQ ID N° 31) gave no PCR product,

The Primers 14 (SEQ ID N° 34) and 15 (SEQ ID N° 35) from the open reading frame of *alarp1* gave a PCR product of 500 bp, *alvepl* (SEQ ID N° 7), which was cloned and sequenced at both strands.

The end primers *of alarp2* (Primers 12 of SEQ ID N° 32 and 13 of SEQ ID N° 33) gave a PCR product of 700 bp, *alvep2* (SEQ ID N° 9), which was cloned and sequenced at both strands.

### Exemple 4 : Expression of AloA-PGPHx in alarp1 transformed BL(21)DE3 E. coli cells

### 4.1. Material and methods:

4.1.1. *Plasmids construction*: the open reading frame of *atarp1* was cloned in the expression vector pRSET( Invitogen) between the restriction sites NdeI and BamHI, and expressed in BL21 cells, provided with the T7 polymerase for high level of expression. Cloning at the NdeI restriction site enables expression of the protein without N-terminal fusion.
   *alarp1* fragment was amplified by PCR. The forward Primer 14 (SEQ ID N° 34) was synthesized with NdeI restriction site and the reverse Primer 15 (SEQ ID N° 35) with BamHI restriction site. The reaction was done with the Expend high Fidelity polymeras kit (Boehringer-Mannheim) at annealing temperature of 55 °C for 25 cycles. The gel purified digested fragment was ligated into the digested and gel purified plasmid vector and transfected by electroporation to JM-109 competent cells.
   The sequence of the cloned construct was confirmed by sequencing both strands.
4.1.2. Protein expression: B1(21)DE3, *E. coli* cells were grown in LB medium-supplemented with 50 µg/ml ampicillin at 37 °C with agitation. At 0.4 O.D, IPTG (isopropylthiogalactoside) (0.4 mM) was added to induce protein production. At 0, 2 and 16 hours samples were taken for protein extraction. Samples from control cells (harbouring the plasmid without the insert) were taken at 16 hours. Proteins were extracted from the cells by freezing and thawing as described (Beeor-Tzahar *et al.,* (1995)*, Febs Letters,* **366,** 151-155). Crude extracts were used for protein analysis on PAGE and activity measurements. Protein concentration was determined using Bio-Rad Protein Assay and bovine y globulin as standard.
   The same extracts were used to determine the activity of AloA-PHGPx (and Cit-PHGPx) toward phosphatidyl-choline hydroperoxide.
4.1.3. PHGPx activity measurements: PHGPx activity was measured as described by Beeor-Tzahar T. *et al.,* (FEBS Letters, (1995), **366,** 151-155).
   The assay is based on the coupled reaction measuring the decrease of NADPH used to reduce GSSG with exogenously supplied glutathione reductase.
   The assay mixture contained 100 mM Tris-HCI buffer, pH=8.2, containing 5 mM EDTA, 15 mM GSH, 0.15 mM NADPH and 3 units of glutathione reductase. 20 µM of phosphatidylcholine-dillinoleoyl-OOH served as the oxidised substrate which was prepared by oxygenation of phosphatidylcholine-dillinoleoyl with lipoxygenase.

### 4.2. Results:

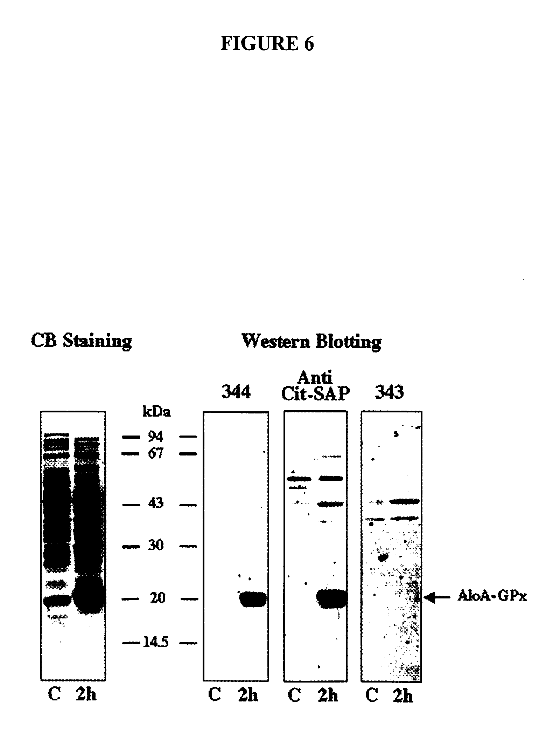

As can be seen from the protein profile in Figure 6, high level of expression was observed after 2 hours of induction with IPTG. About half of the total protein amount is formed by the induced protein.

The 344 antibody specific to the N-terminal of Cit-PHGPx recognized also AloA-PHGPx, although two amino acids are different in the N-terminal peptide against which the antibodies were raised.

The 343 antibody did not recognized AloA-PHGPx (Figure 6).

Determination of the activity of AloA-PHGPx in crude extract obtained from *alarp1*-transfected *E. coli* grown for 2h was 37 units/mg, about the same order of magnitude that was obtained for Cit-PHGPx.

### Example 5 : Engineering of Cit-PHGPx^{SEC} in prokaryotic cells

### 5.1. Materials and Methods:

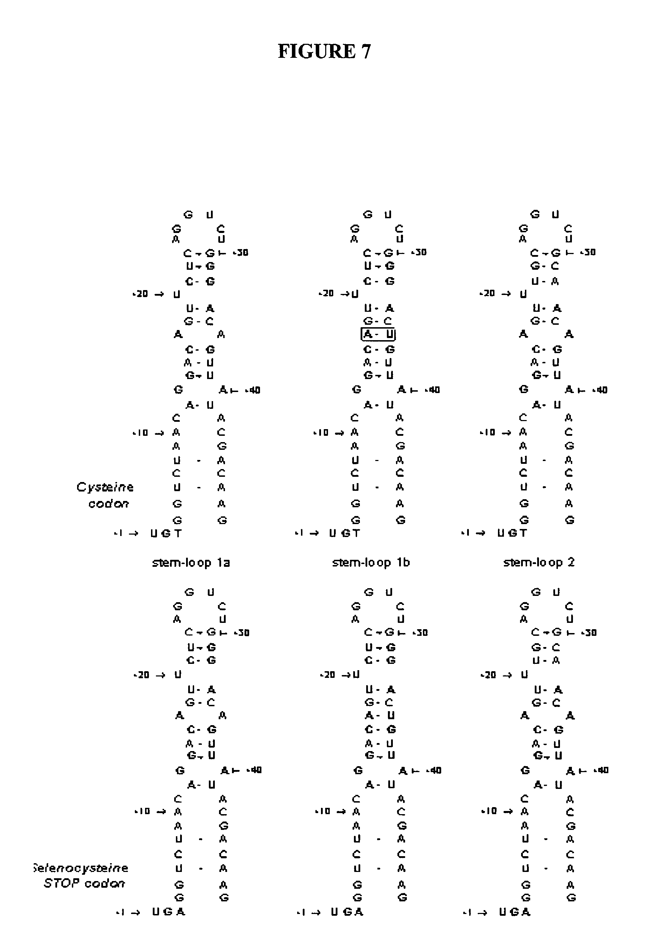

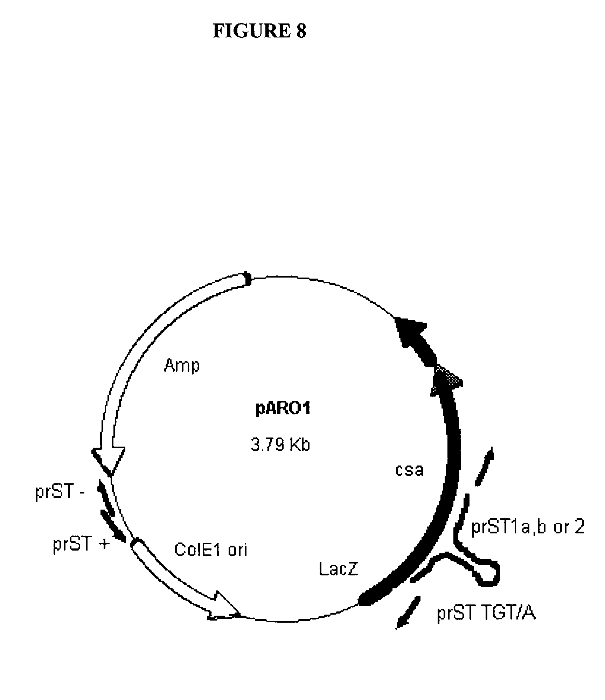

5.1.1. Introduction: Specific insertion of Sec into proteins requires the presence of a UGA codon that determines the position at which Sec is to be inserted, and mRNA stem-loop structure called selenocysteine insertion sequence (SECIS) (Baron *et at,* (1995), in tRNA: Structure, Biosynthesis and Function, 529-544 Stadtman (1996), Annu. Rev. Biochem. **65,** 83-100). As bacterial SECIS is located within the open-reading-frame, immediately adjacent to the 3' side of the UGA codon, the introduction of a stem-loop structure into the mammalian or plant PHGPx mRNA will also result in alteration of the amino acid sequence. However, the domain immediately downstream the catalytic residue of GPx enzymes is rather poorly conserved and then could certainly accommodate some amino acid substitutions. Such plasticity was exploited to design stem-loop structures that could still direct Sec insertion into the Cit-SAP in place of the native cysteine residue.
   The design of mutants was done following analysis of data accumulated on bacterial SECIS.
   Extensive mutational analysis on the SECIS found in the *fdhF* gene, encoding the selenoenzyme formate dehydrogenase H showed that the *E. coli* SECIS must fulfil several requirements (Heider J. *et al.,* (1992) Embo J. **11,** 3759-66 ; Liu Z. *et al.,* (1998), Nucleic Acids Res. **26**, 896-902).
   First, the UGA codon must be in the correct reading frame (Heider J. *et al.,* (1992) reference cited). Second, the distance between the UGA codon and the mini upper stem-loop downstream affects drastically the efficiency of Sec incorporation. In *E. coli fdhF* mRNA, the locus of this mini upper stem-loop was located at a distance of 11 nucleotides from the UGA codon. Increasing the distance by 3 nucleotides resulted in a decrease of Sec incorporation by 50% while increasing by 6 nucleotides the length of the intermediary domain prevented almost totally Sec incorporation (Heider J. *et al.,* (1992) reference cited ; Liu Z. *et al.,* (1998), reference cited).
   Considering the mini upper stem-loop, it has been shown that single changes in the RNA sequence of the loop itself (domain I) abolished Sec incorporation (Heider J. *et al.,* (1992) reference cited ; Liu Z. *et al.,* (1998), reference cited). Indeed, this particular domain has been described to belong to the mRNA motif recognized specifically by the SelB elongation factor (Kromayer M. *et al.,* (1996) J Mol Biol **262,** 413-20). For this reason, no mutations among the 6 nucleotide delimiting the loop were introduced. However, since some of the adjacent base-paired nucleotides (domain II) can be modified without affecting Sec incorporation at the exclusive condition that the base pairing has to be conserved (Liu *et al.,* (1998), reference cited), original mutations were introduced in this domain for the design of mutants with amino acid sequence similar as much as possible to this of Cit-SAP.
5.1.2. Strains and plasmids : *E. coli* DH10B (Life Technologies) was used throughout this study as the host for cloning and expression experiments.
   Plasmid pBluescript SK- was used as the cloning vector for mutagenesis and expression experiments.
   *E.coli* cells were grown in LB medium containing 50 µg of ampicillin per ml. Sodium selenite (Sigma) was added to the growth medium at a final concentration of 5 µM.
   IPTG induction was not necessary because pBluescript is a high copy number plasmid.
5.1.3. Molecular cloning: all recombinant DNA manipulations were carried out by standard procedures. Restriction enzymes were obtained from New England Biolabs and from Boehringer-Mannheim. The Vent DNA polymerase (New England Biolabs) was used for PCR mutagenesis.
5.1.4. Mutagenesis: a serie of plasmids carrying mutated *csa* gene harboring different mRNA stem-loop structures was generated.
   The mutagenesis was done with the PCR technique using the pARO1 plasmid as template. This plasmid carries the *csa* gene that encodes for the Cit-SAP protein (Holland D. *et al.,* (1994) FEBS Lett **337,** 52-5). The required mutations were introduced in the *csa* gene through the primers used during the PCR amplification of the pARO1 plasmid.
   Sequences of the inserted stem-loop structures are described in Figure 7 and sequences of the oligonucleotides used are shown in Table 2.
   The strategy for the mutagenesis of the *csa* gene is presented in Figure 8.
   The stem-loop structure is introduced in the *csa* gene by the mean of two different primers, designated as prST TGT/A and prST 1a/1b/2. Primers prST TGT/A contained the anticodon corresponding to the catalytic residue, either a cysteine (prST TGT = SEQ ID N° 47) or a selenocysteine (prST TGA = SEQ ID N° 48). Primers prST1a/1b/2 coded for the mRNA stem-loop structure. Primers prST+ (SEQ ID N° 46) and prST- (SEQ ID N° 45) hybridized between the ampicillin-resistance gene and the ColEl origin.
   DNA fragments corresponding to PCR amplification of the sequence lying between the primers prST+ and prST TGT/A and between prST- and prsT1a/1b/2, were extracted and purified from agarose gel (QIAEXII extraction kit, QIAGEN). The first fragment was restricted with AlwNI and HpaI enzymes while the second one was only restricted with the A1wNI enzyme while the other extremity remained blunt end. After ligation, the resulting plasmid was transformed in competent cells. Mutated *csa* sequences were verified by DNA sequencing analysis.
5.1.5. Antibodies and protein extracts for immunoblotting :
   In order to test if stem-loops inserted in the *csa* gene were functional, i.e. allowed Sec incorporation, bacteria expressing the different constructs were grown in LB medium complemented or not with 5µM sodium selenite.
   Protein extracts were analysed after SDS-PAGE by staining with Comassie Blue or by staining blotted membranes with red Ponceau followed by immunoblotting analysis with Cit-SAP specific antibodies.

### 5.2. Results:

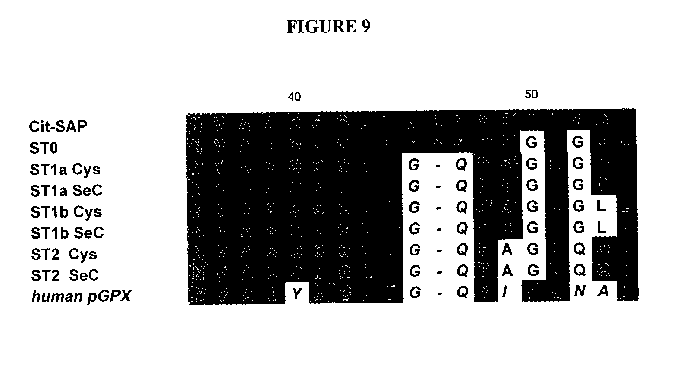

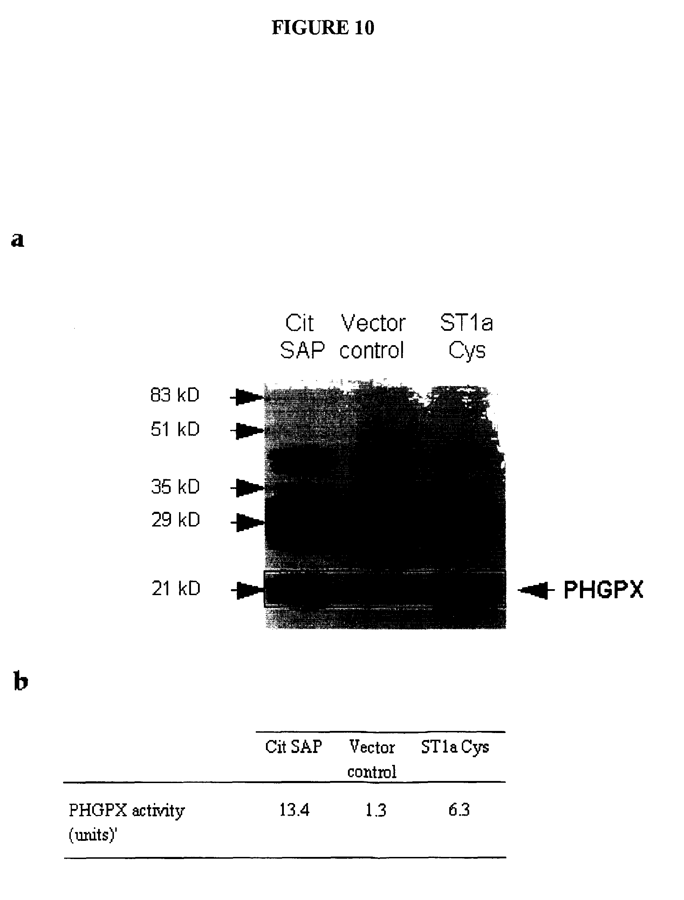

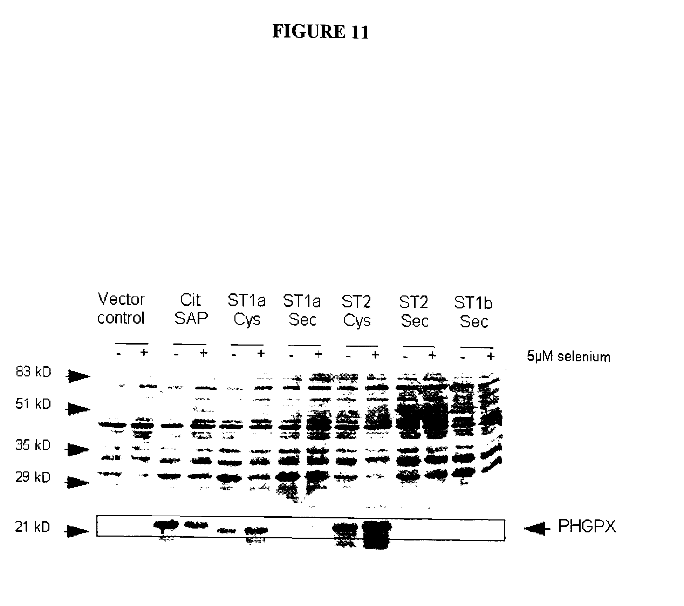

They are given in figures 9, 10 and 11.
5.2.1.Design of the mutants:
By comparing all the possible amino acid sequences constrained by the presence of a stem-loop with that of Cit-SAP, we defined an optimal construct, designated as STO, which imposed a distance of 17 nucleotides between the UGA codon and the stem-loop. This distance was then 6 nucleotides longer than that found in *fdhF* mRNA and consequently 3 nucleotides longer than the longest distance that still permitted Sec incorporation. Since the distance between yhe UGA codon and the stem-loop is critical, the amino acid sequence of the Cit-SAP was shortened by one amino acid.
By using the alignment with mammalian GPx, which contain a selenocysteine in their active site, a deletion in the sequence of many GPx, including the human GPx was observed ; it occurred four residues downstream their native Sec. The deletion environment found in the human GPx was introduced into the Cit-SAP mutants.
Six different mutants have been generated.
Three mutants contained a cysteine residue in their catalytic site. They served to characterize the effect of the mutagenesis on the protein expression and/or stability and on the enzymatic activity compared to those of the Cit-SAP (figure 9).
The other mutants had in their coding sequence a UGA codon that should direct the Sec incorporation. Such mutants permitted first to evaluate the efficiency of Sec incorporation induced by the presence of the modified SECIS and then, to study the effect of the Sec as catalytic residue in the newly synthesized selenoCit-SAPs (figure 9).
For the ST1a mutant, the minimal SECIS, as described by Liu *et al* (1998) (reference cited ) was significantly altered in order to generate a mutant whose amino acid sequence was very homologue to that of the Cit-SAP. The only changes, which are totally unrelated to the PHGPx amino acid sequence, were two glycines in position 50_{E→G} and 52_{S→G}. These mutations leave the upper loop (domain I) of the SECIS identical to that found in the *fdhF* mRNA.
The ST1b mutant differed from the ST1a by only the non base-paired A¹⁸-A³⁵ which was replaced by the complementary bases A¹⁸-U³⁵. This was thought to increase the stability of the stem-loop structure. It resulted in the substitution of the glutamine 53 by a leucine.
Finally, the ST2 mutant was designed to favour the stem-loop recognition by introducing a mRNA structure almost identical to the minimal SECIS with the exception of the base-paired U¹⁹-A²³. For this last construct, three mutations without any homology toward the PHGPx sequences occurred in position 49_{T→A}, 50_{E→G} and 52_{S→Q}.
5.2.2. Expression of mutants containing a cysteine as catalytic residue:
Results are shown in Figure 10.
ST1a-Cys protein migrated slightly more rapidly than the Cit-SAP.
Moreover, coomassie blue analysis of protein extracts revealed that the amount of ST1a-Cys protein in crude extract is significantly lower than that found in the Cit-SAP extract. Indeed, band corresponding to the ST1a-Cys protein presented intensity-about 2 to 4 fold lower than that of the wild-type Cit-SAP (Figure 10a).
Total activity detected in the ST1a-Cys protein extract was 2.4 lower than that measured in the Cit-SAP extract (Figure 10b).
Taken together these results lead to the conclusion that the introduction of SECIS motif in the Cit-SAP coding sequence resulting in the ST1a mutant was not very deleterious for the enzymatic activity.
Relatively high level of protein expression and high PHGPx activity in crude extracts, i.e. similar to that measured with the ST1a-Cys mutant, were as well obtained for the ST1b- and ST2-Cys mutants (data not shown).
5.2.3. Expression of mutants containing a selenocysteine as catalytic residue:
Results are shown in figure 11 and in table 3.
Only enzymes containing a cysteine in their catalytic site were sufficiently overexpressed to be detectable by red Ponceau analysis (Figure 11a). Their expression were totally independent on selenium complementation.
Crude extracts were then analyzed by immunoblotting with Cit-SAP specific antibodies. In these conditions, the addition of sodium selenite into the growth medium induced the expression of two mutants, ST1a-Sec and ST2-Sec (Figure 11b).
This result was confirmed by the PHGPx activity measured in the corresponding crude extracts (Table 3).

**Table 3**

| Mutants | PHGPx activity (units)^{a} | |
|---|---|---|
| | - Selenium | + selenium (5 µM) |
| ST1aCys | 15.6 | n.d. |
| ST1aSec | 2.6 | 10.3 |
| ST1bCys | 14.3 | 16 |
| ST1bSec | 3.7 | 8.8 |
| ST2Cys | 24.2 | 20.7 |
| ST2Sec | 1.9 | 14.2 |

| | | |
|---|---|---|
| ^{a}: a unit is 1 nmole of NADPH oxidized/min at room temperature with respect to total extract protein. | | |

While all protein extracts from bacteria expressing STX-Cys mutants displayed PHGPx activities that ranged around 15-20 units and were not affected by the presence or the absence of sodium selenite in the growth medium (Table 3), STX-Sec crude extracts exhibited high PHGPx activity only if cells were grown in selenium complemented medium.
Thus, it correlated the synthesis of ST1a-Sec and ST2-Sec proteins that the previous immunoblotting revealed (Figure 11b).
The enzymatic activity measured in crude extracts from the STX-Cys and the STX-Sec were on the same order whereas the level of expression of the STX-Cys enzymes was much higher than that of the STX-Sec proteins (Figure 11b and table 3).
Taken together, these results show that specific activities of the STX-Sec enzymes which possess a selenocysteine as catalytic residue, are much higher than that of the corresponding STX-Cys mutants containing in their active site a cysteine.

### Example 6 : Engineering of Cit-PHGPx^{SEC} in eukaryotic cells

### 6.1. Materials and Methods:

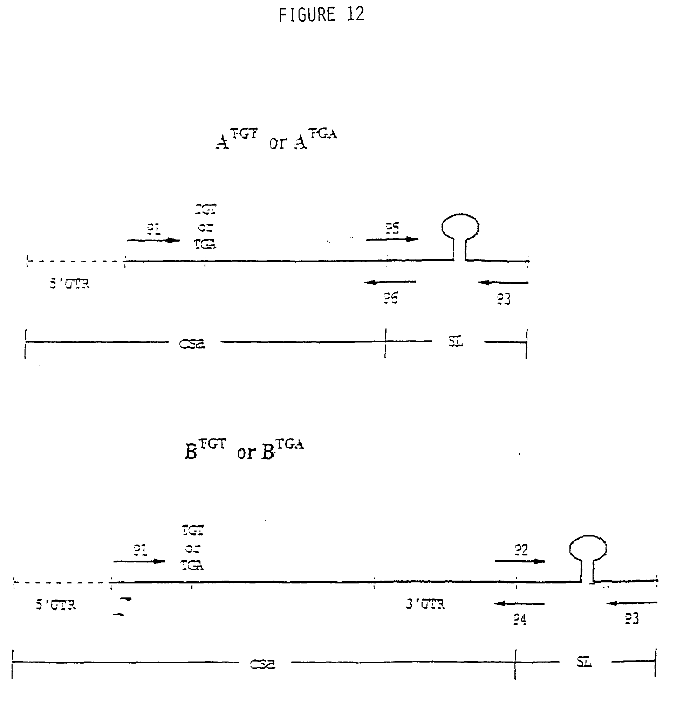

6.1.1. Introduction:
   A selenocysteine residue was introduced in the natural plant PHGPx instead of the cysteine residue located at the active site of the enzyme, by converting the TGT codon to TGA and fusing the 3'UTR of pig PHGPx (Flohé *et al.,* (1994) (reference cited) to the plant gene *(csa)* according to the method described by Leonard *et al.* (J. of Cell Biochem., (1996), **61,** 410-419). The 3'UTR of PHGPx was shown to have a high efficiency SECIS element (Kollmus H. *et al.,* (1996) Nucleic Acids Research. **24:** 1195-1201) which is less influenced from depletion of selenium (Wingler K. *et al.,* (1999) Eur. J. Biochem. **259:** 149-157).
6.1.2. Site directed mutagenesis:
   TGT codon encoding for the amino acid cysteine at position 41 was mutagenized to TGA opal codon. Mutagenesis was carried out by annealing oligo M (Table 4) carrying the mutate nucleotide A, to denature modified pAROl carrying the *csa* gene(Holland D. *et al.,* (1994 ) reference cited).
   T₄ DNA polymerase and T₄ DNA ligase were used to complete the plasmid construction. Modified pAROl was constructed by removing a 0.55 kbp fragment at the fl origin. This modification eliminated a DraIII restriction site, leaving one DraIII restriction site at the site of mutagenesis. Changing the TGT codon to TGA diminished the DraIII restriction site which served for selection of the mutagenized clone.
6.1.3. Synthesis of 3'UTR of pig PHGPx (SL fragment):
   The 3'UTR of pig heart PHGPx (accession No. x76009 nucleotides 555-761) 208 bp long, was synthesized by annealing of 6 synthetic oligonucleotides (Table 4) purchased from Gibco-BRL.
   Three oligonucleotides I-III constructing the 3'→ 5' strand and oligonucleotides IV-VI constructing the complementary strand.
   Oligo IV (SEQ ID N° 55), which is complementary to oligo I (SEQ ID N° 52), has additional 15 nucleotides complementary to oligo II (SEQ ID N° 53), and oligo V (SEQ ID N° 56), which is complementary to oligo II, has additional 15 nucleotides complementary to oligo III (SEQ ID N° 54).
   The restriction site for EcoRI was added to oligo I and VI (SEQ ID N° 57).
   Each pair of complementary oligonucleotides was incubated in 20 µl of water at 95 °C for 5 min and then cooled slowly to 50 °C. Equimolar concentrations of the oligonucleotides were used for each pair, 2 nM for oligo I+IV, 4 nM for oligo II+V and 6 nM for oligo III+VI. The three annealing products were mixed at a ratio of 1:1:1.5, incubated at room temperature for lh and then separated on 3% NuSieve agarose gel (FMC).
   The 200 bp band was purified (Mermaid Kit) and ligated to pUC-19 at the EcoRI site constructing the pUC-SL plasmid. The sequence of all the cloned fragments throughout the work were confirmed by sequencing.
6.1.4. Fusion of the 3'UTR of mammalian PHGPx (SL fragment) to csa:
   Five constructs were prepared :
   - A^{TGT} and A^{TGA} , where the SL fragment was fused to the 3' end of the open reading frame of *csa,*
   - B^{TGT} and B^{TGA} ,where the SL segment was fused to the 3' end of the UTR of *csa* and *csa*^{TGT} containing the open reading frame of *csa* ( Figure 12). The 5'UTR was omitted from A and B constructs.

   Fusion was carried out by PCR in two steps. In the first step an overlapping region of 40 nucleotides was produced between the 3' end of *csa* and the 5' end of SL. Those fragments served as templates in the second PCR reaction constructing the whole A and B fragments.
   The plasmids pARO1 harbouring the native *csa* gene and encoding for the amino acid cysteine (TGT) and the plasmid pARO1mut harbouring the mutated *csa* gene encoding for TGA were used to amplify the *csa* fragment. The plasmid pUC-SL was used to amplify the SL fragment.
   Construction of A^{TGA} was done by amplifying a 500 bp fragment of *csa* with primers P1 (SEQ ID N° 59) and P6 (SEQ ID N° 64), and a 200 bp fragment of SL with primers P3 (SEQ ID N° 61) and P5 (SEQ ID N° 63) (Table 4 and Figure 12). The gel purified fragments were mixed and served as templates for the PCR reaction with primers P1 and P3.
   The construction of B^{TGT} and B^{TGA} were prepared by amplifying a 700 bp fragment of *csa* with primers P1 (SEQ ID N° 59) and P4 (SEQ ID N° 62) and a 200 bp fragment of S L with primers P2 (SEQ ID N° 60) and P3 (SEQ ID N° 61). Again the two fragments were mixed and amplified with primers P1 and P3 ( Table 4 and Figure 12).
   *csa*^{TGT} was constructed by PCR with primers P1 and P7 (SEQ ID N° 65). Primer P1 was synthesized with the EcoRI restriction site and primers P3 and P7 with the XbaI restriction site (Table 4).
   The PCR reactions were carried out with 0.5 µg DNA template and 50 pmoles of primers in 50 µl reaction volume. 15 cycles were done at extension temperature of 40 °C using the tag expender DNA polymerase (Boehringer-Mannheim).
   A^{TGT} was constructed after cloning the constructs in the pYES2 expression vector. The plasmids A^{TGT} and *csa*^{TGT} were cut with SacI, which has a restriction site downstream of the TGT codon and in the cloning region of pYES2. The 200 bp fragment released from *csa*^{TGT} which contains the 5' fragment of *csa*^{TGT} was ligated to the precut A^{TGA} which released this fragment.
6.1.5. Cloning of csa constructs in yeast expression vector and mammalian expression vector
   The constructs A^{TGT}_{'} A^{TGA}, B^{TGT}, B^{TGA} and *csa*^{TGT} were cloned in the yeast expression vector pYES2 (Invitrogen) between the EcoRI and XbaI restriction sites of the polylinker. pYES2 contains the Gall promoter and the URA3 gene for selection.
   Constructs A^{TGT} and A^{TGA} were cloned in the mammalian vector pcDNA3.1 (Invitrogen) and pcDNA3.1His in which a polynucleotide encoding for a polyhistidine tag and a thrombin cleavage site was introduced at the polylinker of pcDNA3.1. A^{TGT} and A^{TGA} were amplified with the forward primer P8 (SEQ ID N° 66) and primer P3 (SEQ ID N° 61). Primer P8 introduced the mammalian transcriptional sequence GCCACC (Kozak (1997), Nucleic Acide Res. **15**, 8125-8148) upstream of the ATG of *csa* and the EcoRI restriction site.
   Cloning in pcDNA3.1His, in frame with the HisTag sequence, was done between the BamHI and XbaI restriction sites of the polylinker. The cloning introduced an additional 17 amino acids at the N-terminal of *csa,* including the polyHisTag and a thrombin cleavage site, two amino acids upstream of the first ATG of *csa.* A^{TGT} and A^{TGA} were amplified with primer P9 (SEQ ID N° 67) which introduced the BamHI restriction site and primer P3 (Table 4).
6.1.6. Expression in yeast cells
   Most of the procedures for yeast propagation were taken from the book « DNA Cloning II » (Romanos M. A. *et al.* (1995) 123-167 Edited by DM Glover and BD Hames, PAS Press).
   Plasmids containing the *csa* constructs were transformed to INVSCI strain of *Saccharomyces cerevisiae* by electroporation. Selection of transformed clones for uracil was done on agar plates, with yeast minimal medium supplemented with 2 % casamino acids, 2 % glucose, 0.02 mg/ml tryptophan. The presence of the plasmid in transformed yeast was confirmed by retransforming *E. coli* cells with DNA extracted from yeast.
   Cells, grown on 2 % raffinose as carbon source, and supplemented with sodium selenite (5 µM) were induced with 2 % galactose, reaching a density of 1 O.D.(optic density). They were harvested after 20 h at 10 O.D.
   Protein extraction was done by mechanical disruption with glass beads. Native extraction was done in the cold room, by vortexing the cells for 7 min with 1 min intervals, in cold extraction buffer containg 100 mM Tris-HCl pH7.5, 1 mM EDTA, supplemented with protease inhibitor cocktail ( AEBSF 2 mM, EDTA 5 mM, leupeptin 1 mM, pepstatin 15 µg/ml, antipain 15 µ/ml, benzamidine 0.1 mM, and aprotinin 10 µg/ml).
   For SDS extraction, cells were boiled in SDS extraction buffer supplemented with the protease inhibitor cocktail, disrupted by vortexing with glass beads and boiled again.
   The yeast extract was separated by a tricine gel system and the protein products were identified with a specific antibody, directed to the N-terminal of *csa*.
6.1.7. Expression in COS cells:
   Expression of *csa* in COS cells was done basically as described by Wen W. *et al.,* (J. Biol. Chem. (1998), **273:** 28533-28541).
   Cells were transformed two days after seeding with 1 µg, 5 µg or 20 µg of plasmid by the DEAE Dextran method. Transformation was carried out with pcDNA3.1 and pcDNA3.1His, both containing A^{TGT} and A^{TGA} constructs. Sodium selenite at concentrations of 5 µg was added to cells at the stage of seeding. Whenever sodium selenite was omitted, an unmeasured concentration of selenium was supplied to the cells by the added medium. Cells were harvested 2 days after transfection and the proteins extracted with lysis buffer (50 mM Tris-HCl, 150 mM NaCI, 10 mM MgCl₂, 5 mM EDTA, 1 % Triton and the proteinase inhibitors :10 µg/ml aprotinin, 20 µg/ml leupeptin and 1 mM PMSF).

### 6.2. Results

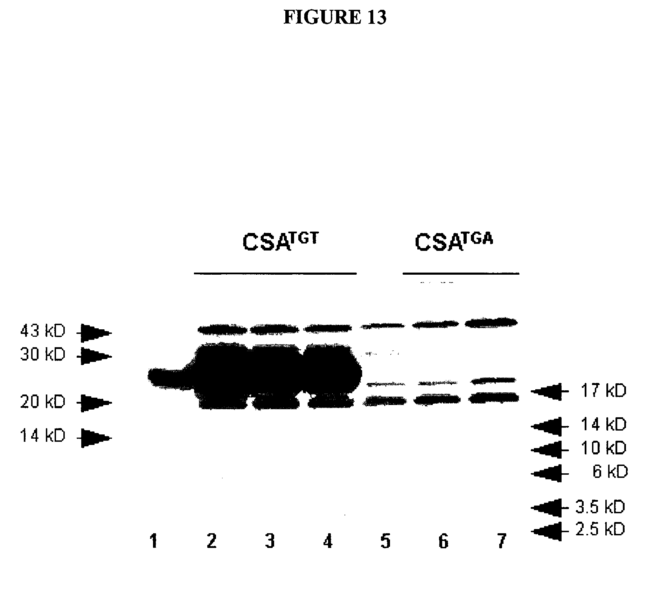

6.2.1. Expression in yeast cells
Results are illustrated in figures 13 and in table 5.
Plant PHGPx could be detected in all the constructs encoding for cysteine and could not be detected in the constructs with the TGA codon, as visualized by SDS-PAGE separation and alkaline phosphatase detection of the Western blots (not shown).
Fusion of the mammalian SECIS element to citrus PHGPx did not interfere with the protein expression. The same amount of expressed protein was detected in all the Cys constructs.
The short peptide (4.5 kD) which might be produced by the termination at the mutated TGA could not be detected after visualization of the blot by ECL (Figure 13).
The expression of the TGT constructs and the absence of any product in the TGA constructs can be readily seen (Figure 13). Overexposure of the blot still did not reveal additional signals (not shown).
A basal level of PHGPx activity of 3 u/mg could be measured in the crude extracts of veast cells harbouring the vector alone (Table 5).

**Table 5**

| | PHGPx activity (units)^{a} |
|---|---|
| vector control pYES2 | 3.3 |
| A^{TGA} | 3.1 |
| B^{TGA} | 2.9 |
| A^{TGT} | 4.8 |
| B^{TGT} | 5.1 |
| csa^{TGT} | 5.5 |

| | |
|---|---|
| ^{a}: a unit is 1 nmole of NADPH oxidized/min at room temperature with respect to total extract protein. | |

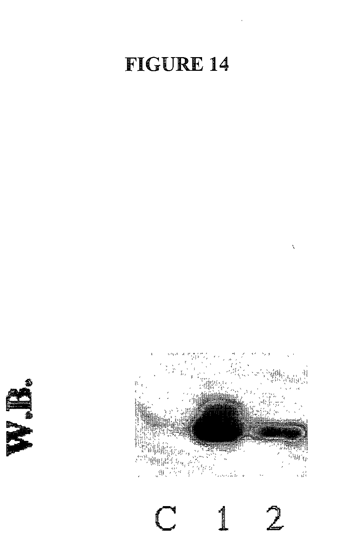

The same activity was measured for the constructs encoding for TGA. Extracts from TGT constructs showed an elevated level of 2 u/mg of activity, which corresponds to about 1.5 % of PHGPx expression (Table 5). This level of expression can be estimated also from the alkaline phosphatase blots.
6.2.2. Expression in COS cells:
Expression of PHGPx^{Cys} and PHGPx^{Sec} could be detected in cells transformed with both pcDNA3.1 and pcDNA3.1His (figure 14). In this specific experiment, selenium was supplied to the cells, only by the serum added to the medium. Due to a long exposure time in the Western-blot membrane in ECL technique, needed for the detection of PHGPx^{Sec}, some cross reaction of the anti-plant PHGPx antibodies with mammalian PHGPx can be seen in the control.

## Claims

1. Isolated plant PHGPx and their analogues comprising an amino acid sequence which is at least 60 % identical or similar to the amino acid sequence of SEQ ID N° 2 over its entire length, with the proviso that they are not SEQ ID N° 2.

2. The isolated plant PHGPx according to claim 1, wherein it is selected from the group consisting of PHGPx isolated from *Aloe arborescens* (SEQ ID N° 4 and SEQ ID N° 6) and PHGPx isolated from *Aloe vera* (SEQ ID N° 8 and SEQ ID N° 10).

3. The analogue of plant PHGPx according to claim 1, which is a recombinant PHGPx wherein the cysteine residue in the active site is replaced by a selenocysteine residue.

4. An analogue according to claim 3 selected from the group consisting of SEQ ID N°11, SEQ ID N°12, SEQ ID N° 13, SEQ ID N° 14, SEQ ID N° 15, SEQ ID N° 16, SEQ ID N° 17, SEQ ID N° 18, SEQ ID N° 19 and SEQ ID N° 20.

5. Isolated PHGPx polynucleotides comprising a sequence which is at least 45 % identical or similar to sequence SEQ ID N° 1 encoding the Cit-SAP sequence of SEQ ID N° 2 over its entire length, with the proviso that these polynucleotides are not SEQ ID N° 1, and their complementary sequences.

6. The polynucleotide according to claim 5 selected from the group consisting of SEQ ID N° 3, SEQ ID N° 5, SEQ ID N° 7, and SEQ ID N° 9.

7. A method for engineering a plant PHGPx containing a selenocysteine instead of a cysteine at its active site, in a prokaryotic cell, wherein introducing an appropriate stem-loop (SECIS) in a gene coding for a plant PHGPx is performed by site specific mutagenesis comprising the steps of:
- performing amplification of the sequence containing said gene coding for a plant PHGPx in two fragments: (i) one fragment amplified by two primers: one, chosen from the sequence coding said gene, containing the anticodon corresponding to the catalytic residue (Cys or Sec), and one located in the plasmid carrying said gene, (ii) one fragment amplified by two primers: one containing the sequence of the stem-loop structure to be introduced and one located in the plasmid carrying said gene,
- digesting the appropriate DNA fragments with appropriate restriction enzymes,
- performing a ligation, and
- transfecting competent prokaryotic cells.

8. A method for engineering according to claim 7, wherein the sequence containing *csa* gene is pARO1 also containing the ampicillin-resistance gene, ColE1 ori and the promoter *lac* and the primers are selected from the group consisting of SEQ ID N° 47 and SEQ ID N° 48 associated to a primer of SEQ ID N° 48 and a primer coding for the mRNA stem-loop structure selected from the group consisting of SEQ ID N° 49, SEQ ID N° 50 and SEQ ID N° 51, said primer being associated to a primer of SEQ ID N° 45, and the digesting step of the first fragment is performed with AlwNI enzyme and HpaI enzyme and the digesting step of the second fragement is performed only with AlwNI enzyme.

9. A method according to anyone of claims 7 and 8, wherein the transfecting step is realised in *E. coli.*

10. A method for engineering plant PHGPx containing a selenocysteine instead of a cysteine at their active site, in an eukaryotic cell, comprising the following steps :
- converting TGT codon to TGA by site directed mutagenesis,
- synthesising of the 3'UTR of pig PHGPx by annealing 6 synthetic oligonucleotides,
- doing fusion of the 3'UTR of pig PHGPx to either the 3' end of the open reading frame of *csa* or the 3' end of *csa,* by PCR,
- cloning in mammalian and yeast vectors and,
- transforming competent eukaryotic cells.

11. A method for engineering according to claim 10 wherein the eukaryotic cells are COS cells.

12. Cosmetic compositions or pharmaceutical dermatological compositions to prevent lipid and phospholipid modification due to their peroxidation, which leads to skin cells damage, ageing and/or necrosis, said compositions comprising at least one active ingredient selected from the group consisting of isolated plant PHGPx or analogues thereof according to anyone of claims 1 to 3, and plant enzymes with PHGPx activity, as active ingredient with antioxidant activity.

13. Cosmetic compositions or pharmaceutical dermatological compositions according to claim 12, wherein the PHGPx is selected from the group consisting of SEQ ID N° 2, SEQ ID N° 4, SEQ ID N° 6, SEQ ID N° 8, SEQ ID N° 9, SEQ ID N° 10, SEQ ID N° 11, SEQ ID N° 12, SEQ ID N° 13, SEQ ID N° 14, SEQ ID N° 15, SEQ ID N° 16, SEQ ID N° 17, SEQ ID N° 18, SEQ ID N° 19 and SEQ ID N° 20 and the plant enzyme with PHGPx activity is gluthatione-S-transferase.

14. Cosmetic compositions or pharmaceutical dermatological compositions according to anyone of claims 12 and 13, further comprising at least an antioxidant selected from the group consisting of Vitamin E, Vitamin C and β-carotene, glutathione and others commonly used antioxidants.

15. The use of compounds selected from the group consisting of plant PHGPx or analogues thereof according to anyone of claims 1 to 4, and from plant enzymes with PHGPx activity or analogues thereof, for the manufacture of an antioxidant cosmetic or pharmaceutical dermatological composition to prevent lipid and phospholipid modification due to their peroxidation, which leads to skin cells damage, ageing and/or necrosis.

16. The use of compounds selected from the group consisting of plant PHGPx or analogues thereof according to anyone of claims 1 to 4, and from plant enzymes with PHGPx activity or analogues thereof, to protect phospholipids used in cosmetic compositions or pharmaceutical dermatological compositions, against phospholipids oxidation.

17. The use of compounds selected from the group consisting of plant PHGPx or analogues thereof according to anyone of claims 1 to 4, and from plant enzymes with PHGPx activity or analogues thereof, as skin-lightening supporting agents in cosmetic compositions or pharmaceutical dermatological compositions.

18. Cosmetic compositions or pharmaceutical dermatological compositions according to anyone of claims 12 to 14, wherein plant-PHGPx or plant enzymes having PHGPx activity are used in the form of enriched plant extracts, partially or completely purified enzyme, recombinant enzyme in prokaryotic or eukaryotic cell types, and genetically engineered modified enzyme.

19. Method of aesthetic treatment of a human to prevent skin cells damage, ageing and/or necrosis, which comprises administering to said human, at least one compound selected from the group consisting of isolated plant PHGPx and analogues thereof, according to claim 1, and of plant enzymes having PHGPx activity, said plant PHGPx being preferably selected from the group consisting of SEQ ID N° 2, SEQ ID N° 4, SEQ ID N° 6, SEQ ID N° 8, SEQ ID N° 9, SEQ ID N° 10, SEQ ID N° 11, SEQ ID N° 12, SEQ ID N° 13, SEQ ID N° 14, SEQ ID N° 15, SEQ ID N° 16, SEQ ID N° 17, SEQ ID N° 18, SEQ ID N° 19 and SEQ ID N° 20 and the plant enzyme with PHGPx activity is gluthatione-S-transferase.


SEQUENCE LISTING

<110> STROSBERG D. A. for and behalf of PLANIR company in formation
<120> PLANT ENZYMES WITH PHOSPHOLIPID HYDROPEROXIDE
      GLUTATHIONE PEROXIDASE ACTIVITY, THEIR ANALOGUES AND
      THEIR USE

<130> s1183EP2

<140>
<141>

<160> 67

<170> PatentIn Ver. 2.1

<210> 1
<211> 862
<212> DNA
<213> Citrus sinensis

<400> 1
atcgcagaac ttccctattt tgacaagaca tttcacttcg aattataagc aaacccttct 60
gagaccttcg aaaagtaatc caatttcttt ggtttcaagg ccttgttttt tcgcttcgag 120
atcggatcat acaatggcaa gccaatcgaa aacttcagtt cacgatttca ctgttaagga 180
cgccaaggga caggatgttg atcttagcat ctacaaggga aagcttcttt tgattgtcaa 240
tgttgcatca cagtgtggct tgaccaattc aaactacact gagctgagtc agttgtacga 300
caagtacaag aatcaaggct tggagattct ggcctttcct tgtaatcagt ttggagctca 360
agagccagga gacaatgaac agattcaaga atttgcatgc actcgcttta aggctgagtt 420
tcccatattt gataaggttg atgttaatgg tgataatgct gctcctttat acaagcatct 480
gaaatccagc aaaggtggac tttttggaga cagcatcaag tggaactttt ccaagttctt 540
ggtggataag gaggggaatg ttgttgagcg ttatgccccc accactagcc ctctgagcat 600
tgagaaggat ataaagaaac ttctggagac tgcttaaccg ctgctacagc ttgcatgatc 660
agcttcttgc taataaaact agagtatcat gctgtataag cactctggtg gaaatatatt 720
gtttagcttt gtctcaatat ctgtagacat ataatgaagt ttcctaatgt cgaagtaaat 780
tcagttgata agtgcttggt ttgtatgtaa tgaatgttcc cagtttttgt aattcaaaat 840
aaaaggtgct tggtgagact ct                                          862


<210> 2
<211> 167
<212> PRT
<213> Citrus sinensis

<400> 2
Met Ala Ser Gln Ser Lys Thr Ser Val His Asp Phe Thr Val Lys Asp
  1               5                  10                  15

Ala Lys Gly Gln Asp Val Asp Leu Ser Ile Tyr Lys Gly Lys Leu Leu
             20                  25                  30

Leu Ile Val Asn Val Ala Ser Gln Cys Gly Leu Thr Asn Ser Asn Tyr
         35                  40                  45

Thr Glu Leu Ser Gln Leu Tyr Asp Lys Tyr Lys Asn Gln Gly Leu Glu
     50                  55                  60

Ile Leu Ala Phe Pro Cys Asn Gln Phe Gly Ala Gln Glu Pro Gly Asp

65                  70                  75                  80

Asn Glu Gln Ile Gln Glu Phe Ala Cys Thr Arg Phe Lys Ala Glu Phe
                85                  90                  95

Pro Ile Phe Asp Lys Val Asp Val Asn Gly Asp Asn Ala Ala Pro Leu
            100                 105                 110

Tyr Lys His Leu Lys Ser Ser Lys Gly Gly Leu Phe Gly Asp Ser Ile
        115                 120                 125

Lys Trp Asn Phe Ser Lys Phe Leu Val Asp Lys Glu Gly Asn Val Val
    130                 135                 140

Glu Arg Tyr Ala Pro Thr Thr Ser Pro Leu Ser Ile Glu Lys Asp Ile
145                 150                 155                 160

Lys Lys Leu Leu Glu Thr Ala
                165

<210> 3
<211> 728
<212> DNA
<213> Aloe arborescens

<220>
<223> alarp 1

<400> 3
ccagtttcag aaacccttct ctctcctcct cgatcagttt cggtaatggc atctcaatca 60
gcaacctcag tccacgattt caccgtcaag gatgctaagg ggaatgatgt ggatctcagt 120
gtttacaaag ggaaggttct gctgattgta aatgttgctt ctaagtgtgg actgactaac 180
tcgaactata cggagctgac tcaattgtac gagaaataca aggatcaagg ttttgagatt 240
ctggctttcc catgcaacca gtttggagca gaagaaccag ggagcaatga ggagattgta 300
gaatttgctt gtacacgctt taaagctgag ttccccatat tcgacaaggt tgatgttaat 360
ggggacaaag ctgcacccat ctacaagttc ttaaagtcca gcaaaggtgg tctgtttggg 420
gatagcatta agtggaactt ctctaagttc ttggtcgaca aggatgggca tgtcgttgac 480
cgttatgcac caaccacatc tcctctgagc attgagaagg acatcaagaa gctattggga 540
gtctcctaaa cttggcgatg cttggacacc taaagttgaa taataaatag atcactggga 600
tcaataggtg cccatctatc ttactgtatg gattcgctat ctatgaggaa tgtcaaagct 660
agtatgagct agaaataaac ttccagttgt gtaataatta gtctctggat gaggttctag 720
tgcttcgt                                                          728

<210> 4
<211> 167
<212> PRT
<213> Aloe arborescens

<220>
<223> PHGPx encoded by alarp 1

<400> 4
Met Ala Ser Gln Ser Ala Thr Ser Val His Asp Phe Thr Val Lys Asp
1               5                   10                  15

Ala Lys Gly Asn Asp Val Asp Leu Ser Val Tyr Lys Gly Lys Val Leu
            20                  25                  30

Leu Ile Val Asn Val Ala Ser Lys Cys Gly Leu Thr Asn Ser Asn Tyr
        35                  40                  45

Thr Glu Leu Thr Gln Leu Tyr Glu Lys Tyr Lys Asp Gln Gly Phe Glu
    50                  55                  60

Ile Leu Ala Phe Pro Cys Asn Gln Phe Gly Ala Glu Glu Pro Gly Ser
65                  70                  75                  80

Asn Glu Glu Ile Val Glu Phe Ala Cys Thr Arg Phe Lys Ala Glu Phe
                85                  90                  95

Pro Ile Phe Asp Lys Val Asp Val Asn Gly Asp Lys Ala Ala Pro Ile
            100                 105                 110

Tyr Lys Phe Leu Lys Ser Ser Lys Gly Gly Leu Phe Gly Asp Ser Ile
        115                 120                 125

Lys Trp Asn Phe Ser Lys Phe Leu Val Asp Lys Asp Gly His Val Val
    130                 135                 140

Asp Arg Tyr Ala Pro Thr Thr Ser Pro Leu Ser Ile Glu Lys Asp Ile
145                 150                 155                 160

Lys Lys Leu Leu Gly Val Ser
                165

<210> 5
<211> 769
<212> DNA
<213> Aloe arborescens

<220>
<223> alarp 2

<400> 5
gcatttcaac cacctctttt tccttctctt cgctgaagcc ttctctcgag atcaatttcg 60
gaaatggctt ctcaatccgc tacatcagtc catgatttca cagtcaagga tgctaaggga 120
aatgatgtgg atctcagtat ttacaagggc aaggttctgc tgattgttaa tgttgcatct 180
cagtgtgggc taacaaattc gaattataca gaattgagtc aactgtacga gaagtacaag 240
gatcaaggtt tcgagatcct ggctttccct tgcaaccagt ttggagctca agaaccagga 300
acgaatgagc agatcctgga atttgcttgc acacgcttca aagctgagta tcccatcttt 360
gacaaggttg atgtgaacgg ggatcaagct gcacccatct acaagttctt gaaatcgagc 420
aaaggtggtt tctttgggga tggcatcaag tggaacttct ctaagttctt ggttgacaag 480
gatggtcatg ttgtggaccg ctatgcaccc actacatctc ctctcagtat tgagaaggat 540
atcaagaagt tattgggagg ttcctaaact tgccgtaacg gagaaggaat gcaaaatgaa 600
taaatcatgg tgattgttta tgttagtact gaagtagtag tactttgtat cccaggtgct 660
tctctttgtt aattggagta ttagttatgg ggattcagta tacactgcta gtctgtggtt 720
taaattactg tgagccatga gaaacaaaag aactatttct gctctcgtg 769

<210> 6
<211> 167

<212> PRT
<213> Aloe arborescens

<220>
<223> PHGPx encoded by alarp 2

<400> 6
Met Ala Ser Gln Ser Ala Thr Ser Val His Asp Phe Thr Val Lys Asp
1               5                   10                  15

Ala Lys Gly Asn Asp Val Asp Leu Ser Ile Tyr Lys Gly Lys Val Leu
            20                  25                  30

Leu Ile Val Asn Val Ala Ser Gln Cys Gly Leu Thr Asn Ser Asn Tyr
        35                  40                  45

Thr Glu Leu Ser Gln Leu Tyr Glu Lys Tyr Lys Asp Gln Gly Phe Glu
    50                  55                  60

Ile Leu Ala Phe Pro Cys Asn Gln Phe Gly Ala Gln Glu Pro Gly Thr
65                  70                  75                  80

Asn Glu Gln Ile Leu Glu Phe Ala Cys Thr Arg Phe Lys Ala Glu Tyr
                85                  90                  95

Pro Ile Phe Asp Lys Val Asp Val Asn Gly Asp Gln Ala Ala Pro Ile
            100                 105                 110

Tyr Lys Phe Leu Lys Ser Ser Lys Gly Gly Phe Phe Gly Asp Gly Ile
        115                 120                 125

Lys Trp Asn Phe Ser Lys Phe Leu Val Asp Lys Asp Gly His Val Val
    130                 135                 140

Asp Arg Tyr Ala Pro Thr Thr Ser Pro Leu Ser Ile Glu Lys Asp Ile
145                 150                 155                 160

Lys Lys Leu Leu Gly Gly Ser
                165

<210> 7
<211> 505
<212> DNA
<213> Aloe vera

<220>
<223> alvep 1

<400> 7
atggcatctc aatcagcaac ctcagtccac gatttcaccg tcaaggatgc taaggggaat 60
gatgtggatc tcagtgttta caaagggaag gttctgctga ttgtaaatgt tgcttctaag 120
tgtggactga ctaactcgaa ctatacggag ctgactcaat tgtacgagaa atacaaggat 180
caaggttttg agattctggc tttcccatgc aaccagtttg gagcagaaga accagggagc 240
aatgaggaga ttgtagaatt tgcttgtaca cgctttaaag ctgagtttcc catattcgac 300
aaggttgatg ttaatgggga ccaagctgca cccatctaca agttcttaaa gtccagcaaa 360
ggtggtctgt ttggggatag cattaagtgg aacttctcta agttcttggt cgacaaggat 420

gggcatgtcg ttgaccgtta tgcaccaacc acacctcctc tgagcattga gaaggacatc 480
aagaagctat tgggagtctc ctaag 505

<210> 8
<211> 167
<212> PRT
<213> Aloe vera

<220>
<223> PHGPx encoded by alvep 1

<400> 8
Met Ala Ser Gln Ser Ala Thr Ser Val His Asp Phe Thr Val Lys Asp
1               5                   10                  15

Ala Lys Gly Asn Asp Val Asp Leu Ser Val Tyr Lys Gly Lys Val Leu
            20                  25                  30

Leu Ile Val Asn Val Ala Ser Lys Cys Gly Leu Thr Asn Ser Asn Tyr
        35                  40                  45

Thr Glu Leu Thr Gln Leu Tyr Glu Lys Tyr Lys Asp Gln Gly Phe Glu
    50                  55                  60

Ile Leu Ala Phe Pro Cys Asn Gln Phe Gly Ala Glu Glu Pro Gly Ser
65                  70                  75                  80

Asn Glu Glu Ile Val Glu Phe Ala Cys Thr Arg Phe Lys Ala Glu Phe
                85                  90                  95

Pro Ile Phe Asp Lys Val Asp Val Asn Gly Asp Gln Ala Ala Pro Ile
            100                 105                 110

Tyr Lys Phe Leu Lys Ser Ser Lys Gly Gly Leu Phe Gly Asp Ser Ile
        115                 120                 125

Lys Trp Asn Phe Ser Lys Phe Leu Val Asp Lys Asp Gly His Val Val
    130                 135                 140

Asp Arg Tyr Ala Pro Thr Thr Pro Pro Leu Ser Ile Glu Lys Asp Ile
145                 150                 155                 160

Lys Lys Leu Leu Gly Val Ser
                165

<210> 9
<211> 736
<212> DNA
<213> Aloe vera

<220>
<223> alvep 2

<400> 9
gcatttcaac cacctctttt tccttctctt cgctgaagcc atctctcgag atcaatttcg 60

ggaatggctt ctcaatccgc tacatcagtc catgatttca cagtcaagga tgctagggga 120
aatgatgtgg atctcagtat ttacaagggc aaggttctgc tgattgttaa tgttgcatct 180
cagtgtgggc taacgaattc gaattataca gaactgagtc aactgtacaa gaagtacaag 240
gatcaaggtt tcgagatcct ggctttccct tgcaaccagt ttggagctca agaaccagga 300
acgaatgagc agatcctgga atttgcttgt acacgcttca aagctgagta tcccatcttt 360
gacaaggttg atgtgaacgg ggatcaagct gcacccatct acaagttctt gaaatcgagc 420
aaaggtggtt tctttgggga tggcatcaag tggaacttct ctaagttctt ggttgacaag 480
gatggtcatg ttgtggaccg ctatgcaccc actacatctc ctctcagtat tgagaaggat 540
atcaagaagt tgttgggagg ttcctaaact tgccataacg gagaaggaat gcaaaataaa 600
tagtaaatga ataaatcatg gtgattgttt atgttagtac agaagtagta gtgctttgta 660
tcccaggtgc ttctctttgt taattggtaa atcactgtga gccatgagaa acaaaagaac 720
tatttctgct ctcgtg 736

<210> 10
<211> 167
<212> PRT
<213> Aloe vera

<220>
<223> PHGPx encoded by alvep 2

<400> 10
Met Ala Ser Gln Ser Ala Thr Ser Val His Asp Phe Thr Val Lys Asp
1               5                   10                  15

Ala Arg Gly Asn Asp Val Asp Leu Ser Ile Tyr Lys Gly Lys Val Leu
            20                  25                  30

Leu Ile Val Asn Val Ala Ser Gln Cys Gly Leu Thr Asn Ser Asn Tyr
        35                  40                  45

Thr Glu Leu Ser Gln Leu Tyr Lys Lys Tyr Lys Asp Gln Gly Phe Glu
    50                  55                  60

Ile Leu Ala Phe Pro Cys Asn Gln Phe Gly Ala Gln Glu Pro Gly Thr
65                  70                  75                  80

Asn Glu Gln Ile Leu Glu Phe Ala Cys Thr Arg Phe Lys Ala Glu Tyr
                85                  90                  95

Pro Ile Phe Asp Lys Val Asp Val Asn Gly Asp Gln Ala Ala Pro Ile
            100                 105                 110

Tyr Lys Phe Leu Lys Ser Ser Lys Gly Gly Phe Phe Gly Asp Gly Ile
        115                 120                 125

Lys Trp Asn Phe Ser Lys Phe Leu Val Asp Lys Asp Gly His Val Val
    130                 135                 140

Asp Arg Tyr Ala Pro Thr Thr Ser Pro Leu Ser Ile Glu Lys Asp Ile
145                 150                 155                 160

Lys Lys Leu Leu Gly Gly Ser
                165

<210> 11
<211> 167
<212> PRT
<213> Artificial Sequence

<220>
<223> Xaa = selenocysteine residue

<220>
<223> Description of Artificial Sequence: Cit-PHGPx
      analog

<400> 11
Met Ala Ser Gln Ser Lys Thr Ser Val His Asp Phe Thr Val Lys Asp
  1               5                  10                  15

Ala Lys Gly Gln Asp Val Asp Leu Ser Ile Tyr Lys Gly Lys Leu Leu
             20                  25                  30

Leu Ile Val Asn Val Ala Ser Gln Xaa Gly Leu Thr Asn Ser Asn Tyr
         35                  40                  45

Thr Glu Leu Ser Gln Leu Tyr Asp Lys Tyr Lys Asn Gln Gly Leu Glu
     50                  55                  60

Ile Leu Ala Phe Pro Cys Asn Gln Phe Gly Ala Gln Glu Pro Gly Asp
 65                  70                  75                  80

Asn Glu Gln Ile Gln Glu Phe Ala Cys Thr Arg Phe Lys Ala Glu Phe
                 85                  90                  95

Pro Ile Phe Asp Lys Val Asp Val Asn Gly Asp Asn Ala Ala Pro Leu
            100                 105                 110

Tyr Lys His Leu Lys Ser Ser Lys Gly Gly Leu Phe Gly Asp Ser Ile
        115                 120                 125

Lys Trp Asn Phe Ser Lys Phe Leu Val Asp Lys Glu Gly Asn Val Val
    130                 135                 140

Glu Arg Tyr Ala Pro Thr Thr Ser Pro Leu Ser Ile Glu Lys Asp Ile
145                 150                 155                 160

Lys Lys Leu Leu Glu Thr Ala
                165

<210> 12
<211> 167
<212> PRT
<213> Artificial Sequence

<220>
<223> Xaa = selenocysteine residue

<220>
<223> Description of Artificial Sequence: PHGPX encoded

by alarp 1

<400> 12
Met Ala Ser Gln Ser Ala Thr Ser Val His Asp Phe Thr Val Lys Asp
1               5                   10                  15

Ala Lys Gly Asn Asp Val Asp Leu Ser Val Tyr Lys Gly Lys Val Leu
            20                  25                  30

Leu Ile Val Asn Val Ala Ser Lys Xaa Gly Leu Thr Asn Ser Asn Tyr
        35                  40                  45

Thr Glu Leu Thr Gln Leu Tyr Glu Lys Tyr Lys Asp Gln Gly Phe Glu
    50                  55                  60

Ile Leu Ala Phe Pro Cys Asn Gln Phe Gly Ala Glu Glu Pro Gly Ser
65                  70                  75                  80

Asn Glu Glu Ile Val Glu Phe Ala Cys Thr Arg Phe Lys Ala Glu Phe
                85                  90                  95

Pro Ile Phe Asp Lys Val Asp Val Asn Gly Asp Lys Ala Ala Pro Ile
            100                 105                 110

Tyr Lys Phe Leu Lys Ser Ser Lys Gly Gly Leu Phe Gly Asp Ser Ile
        115                 120                 125

Lys Trp Asn Phe Ser Lys Phe Leu Val Asp Lys Asp Gly His Val Val
    130                 135                 140

Asp Arg Tyr Ala Pro Thr Thr Ser Pro Leu Ser Ile Glu Lys Asp Ile
145                 150                 155                 160

Lys Lys Leu Leu Gly Val Ser
                165

<210> 13
<211> 167
<212> PRT
<213> Artificial Sequence

<220>
<223> Xaa=selenocysteine residue

<220>
<223> Description of Artificial Sequence: PHGPx encoded
      by alarp 2

<400> 13
Met Ala Ser Gln Ser Ala Thr Ser Val His Asp Phe Thr Val Lys Asp
1               5                   10                  15

Ala Lys Gly Asn Asp Val Asp Leu Ser Ile Tyr Lys Gly Lys Val Leu
            20                  25                  30

Leu Ile Val Asn Val Ala Ser Gln Xaa Gly Leu Thr Asn Ser Asn Tyr

35                  40                  45

Thr Glu Leu Ser Gln Leu Tyr Glu Lys Tyr Lys Asp Gln Gly Phe Glu
    50                  55                  60

Ile Leu Ala Phe Pro Cys Asn Gln Phe Gly Ala Gln Glu Pro Gly Thr
65                  70                  75                  80

Asn Glu Gln Ile Leu Glu Phe Ala Cys Thr Arg Phe Lys Ala Glu Tyr
                85                  90                  95

Pro Ile Phe Asp Lys Val Asp Val Asn Gly Asp Gln Ala Ala Pro Ile
            100                 105                 110

Tyr Lys Phe Leu Lys Ser Ser Lys Gly Gly Phe Phe Gly Asp Gly Ile
        115                 120                 125

Lys Trp Asn Phe Ser Lys Phe Leu Val Asp Lys Asp Gly His Val Val
    130                 135                 140

Asp Arg Tyr Ala Pro Thr Thr Ser Pro Leu Ser Ile Glu Lys Asp Ile
145                 150                 155                 160

Lys Lys Leu Leu Gly Gly Ser
                165

<210> 14
<211> 167
<212> PRT
<213> Artificial Sequence

<220>
<223> Xaa=selenocysteine rsidue

<220>
<223> Description of Artificial Sequence: PHGPX encoded
      by alvep 1

<400> 14
Met Ala Ser Gln Ser Ala Thr Ser Val His Asp Phe Thr Val Lys Asp
  1               5                  10                  15

Ala Lys Gly Asn Asp Val Asp Leu Ser Val Tyr Lys Gly Lys Val Leu
             20                  25                  30

Leu Ile Val Asn Val Ala Ser Lys Xaa Gly Leu Thr Asn Ser Asn Tyr
         35                  40                  45

Thr Glu Leu Thr Gln Leu Tyr Glu Lys Tyr Lys Asp Gln Gly Phe Glu
     50                  55                  60

Ile Leu Ala Phe Pro Cys Asn Gln Phe Gly Ala Glu Glu Pro Gly Ser
 65                  70                  75                  80

Asn Glu Glu Ile Val Glu Phe Ala Cys Thr Arg Phe Lys Ala Glu Phe
                 85                  90                  95

Pro Ile Phe Asp Lys Val Asp Val Asn Gly Asp Gln Ala Ala Pro Ile
            100                 105                 110

Tyr Lys Phe Leu Lys Ser Ser Lys Gly Gly Leu Phe Gly Asp Ser Ile
        115                 120                 125

Lys Trp Asn Phe Ser Lys Phe Leu Val Asp Lys Asp Gly His Val Val
    130                 135                 140

Asp Arg Tyr Ala Pro Thr Thr Pro Pro Leu Ser Ile Glu Lys Asp Ile
145                 150                 155                 160

Lys Lys Leu Leu Gly Val Ser
                165

<210> 15
<211> 167
<212> PRT
<213> Artificial Sequence

<220>
<223> Xaa=selenocysteine residue

<220>
<223> Description of Artificial Sequence: PHGPx encoded
by alvep 2

<400> 15
Met Ala Ser Gln Ser Ala Thr Ser Val His Asp Phe Thr Val Lys Asp
1               5                   10                  15

Ala Arg Gly Asn Asp Val Asp Leu Ser Ile Tyr Lys Gly Lys Val Leu
            20                  25                  30

Leu Ile Val Asn Val Ala Ser Gln Xaa Gly Leu Thr Asn Ser Asn Tyr
        35                  40                  45

Thr Glu Leu Ser Gln Leu Tyr Lys Lys Tyr Lys Asp Gln Gly Phe Glu
    50                  55                  60

Ile Leu Ala Phe Pro Cys Asn Gln Phe Gly Ala Gln Glu Pro Gly Thr
65                  70                  75                  80

Asn Glu Gln Ile Leu Glu Phe Ala Cys Thr Arg Phe Lys Ala Glu Tyr
                85                  90                  95

Pro Ile Phe Asp Lys Val Asp Val Asn Gly Asp Gln Ala Ala Pro Ile
            100                 105                 110

Tyr Lys Phe Leu Lys Ser Ser Lys Gly Gly Phe Phe Gly Asp Gly Ile
        115                 120                 125

Lys Trp Asn Phe Ser Lys Phe Leu Val Asp Lys Asp Gly His Val Val
    130                 135                 140

Asp Arg Tyr Ala Pro Thr Thr Ser Pro Leu Ser Ile Glu Lys Asp Ile
145                 150                 155                 160

Lys Lys Leu Leu Gly Gly Ser
                165

<210> 16
<211> 166
<212> PRT
<213> Artificial Sequence

<220>
<223> Xaa=selenocysteine residue

<220>
<223> Description of Artificial Sequence: ST1aSec mutant

<400> 16
Met Ala Ser Gln Ser Lys Thr Ser Val His Asp Phe Thr Val Lys Asp
  1               5                  10                  15

Ala Lys Gly Gln Asp Val Asp Leu Ser Ile Tyr Lys Gly Lys Leu Leu
             20                  25                  30

Leu Ile Val Asn Val Ala Ser Gln Xaa Gly Leu Thr Gly Gln Phe Ser
         35                  40                  45

Gly Leu Gly Gln Leu Tyr Asp Lys Tyr Lys Asn Gln Gly Leu Glu Ile
     50                  55                  60

Leu Ala Phe Pro Cys Asn Gln Phe Gly Ala Gln Glu Pro Gly Asp Asn
 65                  70                  75                  80

Glu Gln Ile Gln Glu Phe Ala Cys Thr Arg Phe Lys Ala Glu Phe Pro
                 85                  90                  95

Ile Phe Asp Lys Val Asp Val Asn Gly Asp Asn Ala Ala Pro Leu Tyr
            100                 105                 110

Lys His Leu Lys Ser Ser Lys Gly Gly Leu Phe Gly Asp Ser Ile Lys
        115                 120                 125

Trp Asn Phe Ser Lys Phe Leu Val Asp Lys Glu Gly Asn Val Val Glu
    130                 135                 140

Arg Tyr Ala Pro Thr Thr Ser Pro Leu Ser Ile Glu Lys Asp Ile Lys
145                 150                 155                 160

Lys Leu Leu Glu Thr Ala
                165

<210> 17
<211> 166
<212> PRT

<213> Artificial Sequence

<220>
<223> Xaa=selenocysteine residue

<220>
<223> Description of Artificial Sequence: ST1bSec mutant

<400> 17
Met Ala Ser Gln Ser Lys Thr Ser Val His Asp Phe Thr Val Lys Asp
1               5                   10                  15

Ala Lys Gly Gln Asp Val Asp Leu Ser Ile Tyr Lys Gly Lys Leu Leu
            20                  25                  30

Leu Ile Val Asn Val Ala Ser Gln Xaa Gly Leu Thr Gly Gln Phe Ser
        35                  40                  45

Gly Leu Gly Leu Leu Tyr Asp Lys Tyr Lys Asn Gln Gly Leu Glu Ile
    50                  55                  60

Leu Ala Phe Pro Cys Asn Gln Phe Gly Ala Gln Glu Pro Gly Asp Asn
65                  70                  75                  80

Glu Gln Ile Gln Glu Phe Ala Cys Thr Arg Phe Lys Ala Glu Phe Pro
                85                  90                  95

Ile Phe Asp Lys Val Asp Val Asn Gly Asp Asn Ala Ala Pro Leu Tyr
            100                 105                 110

Lys His Leu Lys Ser Ser Lys Gly Gly Leu Phe Gly Asp Ser Ile Lys
        115                 120                 125

Trp Asn Phe Ser Lys Phe Leu Val Asp Lys Glu Gly Asn Val Val Glu
    130                 135                 140

Arg Tyr Ala Pro Thr Thr Ser Pro Leu Ser Ile Glu Lys Asp Ile Lys
145                 150                 155                 160

Lys Leu Leu Glu Thr Ala
                165

<210> 18
<211> 166
<212> PRT
<213> Artificial Sequence

<220>
<223> Xaa=selenocysteine residue

<220>
<223> Description of Artificial Sequence: ST2Sec mutant
      ; analog of PHGPx encoded by alarp 1

<400> 18
Met Ala Ser Gln Ser Ala Thr Ser Val His Asp Phe Thr Val Lys Asp

1               5                   10                  15

Ala Lys Gly Asn Asp Val Asp Leu Ser Val Tyr Lys Gly Lys Val Leu
            20                  25                  30

Leu Ile Val Asn Val Ala Ser Lys Xaa Gly Leu Thr Gly Gln Phe Ala
        35                  40                  45

Gly Leu Gln Gln Leu Tyr Glu Lys Tyr Lys Asp Gln Gly Phe Glu Ile
    50                  55                  60

Leu Ala Phe Pro Cys Asn Gln Phe Gly Ala Glu Glu Pro Gly Ser Asn
 65                 70                  75                  80

Glu Glu Ile Val Glu Phe Ala Cys Thr Arg Phe Lys Ala Glu Phe Pro
                85                  90                  95

Ile Phe Asp Lys Val Asp Val Asn Gly Asp Lys Ala Ala Pro Ile Tyr
            100                 105                 110

Lys Phe Leu Lys Ser Ser Lys Gly Gly Leu Phe Gly Asp Ser Ile Lys
        115                 120                 125

Trp Asn Phe Ser Lys Phe Leu Val Asp Lys Asp Gly His Val Val Asp
    130                 135                 140

Arg Tyr Ala Pro Thr Thr Ser Pro Leu Ser Ile Glu Lys Asp Ile Lys
145                 150                 155                 160

Lys Leu Leu Gly Val Ser
                165

<210> 19
<211> 166
<212> PRT
<213> Artificial Sequence

<220> [?]
<223> Xaa=selenocysteine residue

<220>
<223> Description of Artificial Sequence: ST2 Sec mutant
      ; analog of PHGPx encoded by alvep 2

<400> 19
Met Ala Ser Gln Ser Ala Thr Ser Val His Asp Phe Thr Val Lys Asp
  1               5                   10                  15

Ala Arg Gly Asn Asp Val Asp Leu Ser Ile Tyr Lys Gly Lys Val Leu
            20                  25                  30

Leu Ile Val Asn Val Ala Ser Gln Xaa Gly Leu Thr Gly Gln Phe Ala
        35                  40                  45

Gly Leu Gln Gln Leu Tyr Lys Lys Tyr Lys Asp Gln Gly Phe Glu Ile
    50                  55                  60

Leu Ala Phe Pro Cys Asn Gln Phe Gly Ala Gln Glu Pro Gly Thr Asn
65                  70                  75                  80

Glu Gln Ile Leu Glu Phe Ala Cys Thr Arg Phe Lys Ala Glu Tyr Pro
                85                  90                  95

Ile Phe Asp Lys Val Asp Val Asn Gly Asp Gln Ala Ala Pro Ile Tyr
            100                 105                 110

Lys Phe Leu Lys Ser Ser Lys Gly Gly Phe Phe Gly Asp Gly Ile Lys
        115                 120                 125

Trp Asn Phe Ser Lys Phe Leu Val Asp Lys Asp Gly His Val Val Asp
    130                 135                 140

Arg Tyr Ala Pro Thr Thr Ser Pro Leu Ser Ile Glu Lys Asp Ile Lys
145                 150                 155                 160

Lys Leu Leu Gly Gly Ser
                165

<210> 20
<211> 166
<212> PRT
<213> Artificial Sequence

<220>
<223> Xaa=selenocysteine residue

<220>
<223> Description of Artificial Sequence: ST2 mutant

<400> 20
Met Ala Ser Gln Ser Lys Thr Ser Val His Asp Phe Thr Val Lys Asp
  1               5                  10                  15

Ala Lys Gly Gln Asp Val Asp Leu Ser Ile Tyr Lys Gly Lys Leu Leu
             20                  25                  30

Leu Ile Val Asn Val Ala Ser Gln Xaa Gly Leu Thr Gly Gln Phe Ala
         35                  40                  45

Gly Leu Gln Gln Leu Tyr Asp Lys Tyr Lys Asn Gln Gly Leu Glu Ile
     50                  55                  60

Leu Ala Phe Pro Cys Asn Gln Phe Gly Ala Gln Glu Pro Gly Asp Asn
 65                  70                  75                  80

Glu Gln Ile Gln Glu Phe Ala Cys Thr Arg Phe Lys Ala Glu Phe Pro
                 85                  90                  95

Ile Phe Asp Lys Val Asp Val Asn Gly Asp Asn Ala Ala Pro Leu Tyr
            100                 105                 110

Lys His Leu Lys Ser Ser Lys Gly Gly Leu Phe Gly Asp Ser Ile Lys

115                 120                 125

Trp Asn Phe Ser Lys Phe Leu Val Asp Lys Glu Gly Asn Val Val Glu
    130                 135                 140

Arg Tyr Ala Pro Thr Thr Ser Pro Leu Ser Ile Glu Lys Asp Ile Lys
145                 150                 155                 160

Lys Leu Leu Glu Thr Ala
                165

<210> 21
<211> 17
<212> DNA
<213> Artificial Sequence

<220>
<223> Description of Artificial Sequence: primer

<400> 21
gttttcccag tcacgag                                                17

<210> 22
<211> 17
<212> DNA
<213> Artificial Sequence

<220>
<223> Description of Artificial Sequence: primer

<400> 22
gttttcccag tcacgag                                                17

<210> 23
<211> 23
<212> DNA
<213> Artificial Sequence

<220>
<223> Description of Artificial Sequence: primer

<400> 23
gtnaangtng cntcnnantg ngg                                         23

<210> 24
<211> 25
<212> DNA
<213> Artificial Sequence

<220>
<223> Description of Artificial Sequence: primer

<400> 24

gaacttgtag atgggtgcag ctttg 25

<210> 25
<211> 25
<212> DNA
<213> Artificial Sequence

<220>
<223> Description of Artificial Sequence: primer

<400> 25
ctatcgattc tggaaccttc agagg 25

<210> 26
<211> 25
<212> DNA
<213> Artificial Sequence

<220>
<223> Description of Artificial Sequence: primer

<400> 26
cctctgaagg ttccagaatc gatag 25

<210> 27
<211> 25
<212> DNA
<213> Artificial Sequence

<220>
<223> Description of Artificial Sequence: primer

<400> 27
cttctgctcc aaactggttg catgg 25

<210> 28
<211> 25
<212> DNA
<213> Artificial Sequence

<220>
<223> Description of Artificial Sequence: primer

<400> 28
gaagcgtgtg caagcaaatt ccagg 25

<210> 29
<211> 25
<212> DNA
<213> Artificial Sequence

<220>
<223> Description of Artificial Sequence: primer

<400> 29
ggttcttgag ctccaaactg gttgc                                          25

<210> 30
<211> 21
<212> DNA
<213> Artificial Sequence

<220>
<223> Description of Artificial Sequence: primer

<400> 30
ccagtttcag aaacccttct c                                              21

<210> 31
<211> 22
<212> DNA
<213> Artificial Sequence

<220>
<223> Description of Artificial Sequence: primer

<400> 31
acgaagcact agaacctcat cc                                             22

<210> 32
<211> 23
<212> DNA
<213> Artificial Sequence

<220>
<223> Description of Artificial Sequence: primer

<400> 32
gcatttcaac cacctctttt tcc                                            23

<210> 33
<211> 20
<212> DNA
<213> Artificial Sequence

<220>
<223> Description of Artificial Sequence: primer

<400> 33
cacgagagca gaaatagttc                                                20

<210> 34
<211> 32
<212> DNA
<213> Artificial Sequence

<220>
<223> Description of Artificial Sequence: primer

<400> 34
cctggaagca tatggcatct caatcagcaa cc                                32


<210> 35
<211> 28
<212> DNA
<213> Artificial Sequence

<220>
<223> Description of Artificial Sequence: primer

<400> 35
cgtggatcct taggagactc ccaatagc                                     28


<210> 36
<211> 18
<212> DNA
<213> Artificial Sequence

<220>
<223> Description of Artificial Sequence: primer

<400> 36
gctgcaccca tctacaag                                                18


<210> 37
<211> 27
<212> DNA
<213> Artificial Sequence

<220>
<223> Description of Artificial Sequence: primer

<400> 37
ccatcctaat acgactcact atagggc                                      27


<210> 38
<211> 23
<212> DNA
<213> Artificial Sequence

<220>
<223> Description of Artificial Sequence: primer

<400> 38
actcactata gggctcgagc ggc                                          23


<210> 39
<211> 638
<212> DNA

<213> Aloe arborescens

<400> 39
gtgaacgtgg cgtcgcagtg aggactgact aactcgaact atacggagct gactcaattg 60
tacgagaaat acaaggatca aggttttgag attctggctt tcccatgcaa ccagtttgga 120
gcagaagaac cagggagcaa tgaggagatt gtagaatttg cttgtacacg ctttaaagct 180
gagtttccca tattcgacaa ggttgatgtt aatggggaca aagctgcacc catctacaag 240
ttcttaaagt ccagcaaagg tggtctgttt ggggatagca ttaagtggaa cttctctaag 300
ttcttggtcg acaaggatgg gcatgtcgtt gaccgttatg caccaaccac atctcctctg 360
agcattgaga aggacatcaa gaagctattg ggagtctcct aaacttggcg atgcttggac 420
acctaaagtt gaataataaa tagatcactg ggatcaatag gtgcccatct atcttactgt 480
atggattcgc tatctatgag gaatgtcaaa gctagtatga gctagaaata aacttccagt 540
tgtgtaataa ttagtctctg gatgaggttc tagtgcttcg ttatgtccga ggtattgtat 600
cctagtgctt tgttataatc gtaaaaaaaa aaaaaaaa 638

<210> 40
<211> 673
<212> DNA
<213> Aloe arborescens

<400> 40
gtgaacgtgg cgtcgcagtg agggctaacg aattcgaatt atacagaatt gagtcaactg 60
tacgagaagt acaaggatca aggtttcgag atcctggctt tcccttgcaa ccagtttgga 120
gctcaagaac caggaacgaa tgagcagatc ctggaatttg cttgcacacg cttcaaagct 180
gagtatccca tctttgacaa ggttgatgtg aatggggatc aaggtgcacc catctacaag 240
ttcttgaaat cgagcaaagg tggtttcttt ggggatggca tcaagtggaa cttctctaag 300
ttcttggttg acaaggatgg tcatgttgtg gaccgctatg cgcccactac atctcctctc 360
agtattgaga aggatatcaa gaagttgttg ggaggttcct aaacttgcca taacggagaa 420
ggaatgcaaa ataaataaat gaataaatca tggtgattgt ttatgttagt actgaagtag 480
tagtgctttg tatcccaggt gtttctcttt gttaattgga gtattagtta tggggattca 540
gtatatactg ctagtctgtg gtttaaaata ctgagccatg agaaacaaaa gaactatttc 600
tgctctcgtg agattggtaa caccatgaaa tttccgatga atattgataa tgatttcgac 660
caaaaaaaaa aaa 673

<210> 41
<211> 699
<212> DNA
<213> Aloe arborescens

<400> 41
tgaacgtggc gtcgcagtgc gggctaacga attcgaatta tacagaattg agtcaactgt 60
acgagaagta caaggatcaa ggtttcgaga tcctggcttt cccttgcaac cagtttggag 120
ctcaagaacc aggaacgaat gagcagatcc tggaatttgc ttgcacacgc ttcaaagctg 180
agtatcccat ctttgacaag gttgatgtga atggggatca agctgcaccc atctacaagt 240
tcttgaaatc gagcaaaggt ggtttctttg gggatggcat caagtggaac ttctctaagt 300
tcttggttga caaggatggt catgttgtgg accgctatgc acccactaca tctcctctca 360
gtattgagaa ggatatcaag aagttattgg gaggttccta aacttgccgt aacggagaag 420
gaatgcaaaa tgaataaatc atggtgattg tttatgttag tacttgaagt agtagtactt 480
tgtatcccag gtgnttctct ttgtnaattg gagtattagt tatggggatt cagtatacac 540
tgntagtctg tggtttnaat tantgtgagc catgagaaac aaaagaacta tttctgctct 600
cgtgggattg gtaacaccat gaaatttcgg atgaatattg ataatgattt cgaccacgaa 660
tgtgcgcctt ggaggagcat tgctaaaaaa aaaaaaaaa 699

<210> 42
<211> 670

<212> DNA
<213> Aloe arborescens

<400> 42
tgtgaacgtg gcgtcgaaat gagggctaac aaattcgaat tatacagaat tgagtcaact 60
gtacgagaag tacaaggatc aaggtttcga gatcctggct ttcccttgca accagtttgg 120
agctcaagaa ccaggaacga atgagcagat cctggaattt gcttgcacac gcttcaaagc 180
tgagtatccc atctttgaca aggttgatgt gaacggggat caagctgcac ccatctacaa 240
gttcttgaaa tcgagcaaag gtggtttctt tggggatggc atcaagtgga acttctctaa 300
gttcttggtt gacaaggatg gtcatgttgt ggaccgctat gcacccacta catctcctct 360
cagtattgag aaggatatca agaagttatt gggaggttcc taaacttgcc gtaacggaga 420
aggaatgcaa aatgaataaa tcatggtgat tgtttatgtt agtacttgaa gtagtagtac 480
tttgtatccc aggtgcttct ctttgttaat tggagtatta gttatgggga ttcagtatac 540
actgctagtc tgtggtttaa attactgtga gccatgagaa acaaaagaac tatttctgct 600
ctcgtgggat tggtaacacc atgaaatttc ggatgaatat tgataatgat ttcgaccaaa 660
aaaaaaaaaa 670

<210> 43
<211> 274
<212> DNA
<213> Aloe arborescens

<400> 43
ccagtttcag aaacccttct ctttcttcct cgatcggttt cggtaatggc atctcaatca 60
gcaacctcag accacgattt caccgtcaag gatgctaagg gggatgatgt ggatctcagt 120
gtttacaaag ggaaggttct gctgattgta aatgttgctt ctaagtgtgg actgactaac 180
tcgaactata cggagctgac tcaattgtac gagaaataca aggatcaagg ttttgagatt 240
ctggctttcc catgcaacca gtttggagca gaag 274

<210> 44
<211> 297
<212> DNA
<213> Aloe arborescens

<400> 44
cgcatttcaa ccacctcttt ttccttctct tcgctgaagc cttctctcga gatcaatttc 60
ggaaatggct tctcaatccg ctacatcagt ccatgatttc acagtcaagg atgctaaggg 120
aaatgatgtg gatctcagta tttacaaggg caaggttctg ctgattgtta atgttgcatc 180
tcagtgtggg ctaacaaatt cgaattatac agaattgagt caactgtacg agaagtacaa 240
ggatcaaggt ttcgagatcc tggctttccc ttgcaaccag tttggagctc aagaacc 297

<210> 45
<211> 22
<212> DNA
<213> Artificial Sequence

<220>
<223> Description of Artificial Sequence: primers

<400> 45
tacgcagcag ccactggtaa ca 22

<210> 46
<211> 18

<212> DNA
<213> Artificial Sequence

<220>
<223> Description of Artificial Sequence: primers

<400> 46
tcctgttacc agtggctg                                                    18


<210> 47
<211> 45
<212> DNA
<213> Artificial Sequence

<220>
<223> Description of Artificial Sequence: primers

<400> 47
aaagctgtta actgtcctgt tagaccacac tgtgatgcaa cattg                      45


<210> 48
<211> 45
<212> DNA
<213> Artificial Sequence

<220>
<223> Description of Artificial Sequence: primers

<400> 48
aaagctgtta actgtcctgt tagacctcac tgtgatgcaa cattg                      45


<210> 49
<211> 34
<212> DNA
<213> Artificial Sequence

<220>
<223> Description of Artificial Sequence: primers

<400> 49
ctcaggtctg ggacagttat acgacaagta caag                                  34


<210> 50
<211> 34
<212> DNA
<213> Artificial Sequence

<220>
<223> Description of Artificial Sequence: primers

<400> 50
ctcaggtctg ggactgttat acgacaagta caag                                  34

<210> 51
<211> 34
<212> DNA
<213> Artificial Sequence

<220>
<223> Description of Artificial Sequence: primers

<400> 51
tgcaggtctg caacagttat acgacaagta caag                                34


<210> 52
<211> 74
<212> DNA
<213> Artificial Sequence

<220>
<223> Description of Artificial Sequence: primers

<400> 52
aattcctcaa caagtgtgtg ctgagcctgc tgcctgtgcc ctcagagcct tccacctggc 60
acccatgaca gtct                                                      74


<210> 53
<211> 79
<212> DNA
<213> Artificial Sequence

<220>
<223> Description of Artificial Sequence: primers

<400> 53
gcctaaaaac cagcccctgg tggggcagac tcgagaacct ggcgtgcaac cccgccagag 60
gaaggtccct tggcccagc                                                 79


<210> 54
<211> 61
<212> DNA
<213> Artificial Sequence

<220>
<223> Description of Artificial Sequence: primers

<400> 54
ttgcagctcg caccccaccc caggctgcct tgtgggaata aaccatacaa attggctaaa 60
g                                                                    61


<210> 55
<211> 85
<212> DNA
<213> Artificial Sequence

<220>
<223> Description of Artificial Sequence: primers

<400> 55
ggctggtttt taggcagact gtcatgggtg ccaggtggaa ggctctgagg gcacaggcag 60
caggctcagc acacacttgt tgagg 85

<210> 56
<211> 79
<212> DNA
<213> Artificial Sequence

<220>
<223> Description of Artificial Sequence: primers

<400> 56
gggtgcgagc tgcaagctgg gccaagggac cttcctctgg cggggttgca cgccaggttc 60
tcgagtctgc cccaccagg 79

<210> 57
<211> 50
<212> DNA
<213> Artificial Sequence

<220>
<223> Description of Artificial Sequence: primers

<400> 57
aattctttag ccaatttgta tggtttattc ccacaaggca gcctggggtg 50

<210> 58
<211> 17
<212> DNA
<213> Artificial Sequence

<220>
<223> Description of Artificial Sequence: primers

<400> 58
tcacagtgag gcttgac 17

<210> 59
<211> 41
<212> DNA
<213> Artificial Sequence

<220>
<223> Description of Artificial Sequence: primers

<400> 59
atccatatca gaattcataa aatggcaagc caatcgaaaa c 41

<210> 60
<211> 40
<212> DNA

<213> Artificial Sequence

<220>
<223> Description of Artificial Sequence: primers

<400> 60
aaggtgcttg gtgagactct ctcaacaagt gtgtgctgag                                40


<210> 61
<211> 34
<212> DNA
<213> Artificial Sequence

<220>
<223> Description of Artificial Sequence: primers

<400> 61
atggatatga tctagattta gccaatttgt atgg                                      34


<210> 62
<211> 38
<212> DNA
<213> Artificial Sequence

<220>
<223> Description of Artificial Sequence: primers

<400> 62
ctcagcacac acttgttgag agagtctcac caagcacc                                  38


<210> 63
<211> 40
<212> DNA
<213> Artificial Sequence

<220>
<223> Description of Artificial Sequence: primers

<400> 63
aacttctgga gactgcttaa ctcaacaagt gtgtgctgag                                40


<210> 64
<211> 38
<212> DNA
<213> Artificial Sequence

<220>
<223> Description of Artificial Sequence: primers

<400> 64
ctcagcacac acttgttgag ttaagcagtc tccagaag                                  38


<210> 65

<211> 34
<212> DNA
<213> Artificial Sequence

<220>
<223> Description of Artificial Sequence: primers

<400> 65
atggatatga tctagattaa gcagtctcca gaag 34

<210> 66
<211> 36
<212> DNA
<213> Artificial Sequence

<220>
<223> Description of Artificial Sequence: primers

<400> 66
ctggaattcg ccaccaatgg caagccaatc gaaaac 36

<210> 67
<211> 30
<212> DNA
<213> Artificial Sequence

<220>
<223> Description of Artificial Sequence: primers

<400> 67
acgaggatcc atggcaagcc aatcgaaaac 30